# EUROPEAN PATENT SPECIFICATION

(11) **EP 4 359 786 B1**
(45) Date of publication and mention of the grant of the patent: **16.09.2026**
(21) Application number: 22744874.3
(22) Date of filing: 22.06.2022
(51) Int. Cl.: G01N 30/34, A61K 39/395, B01D 15/16, B01D 15/36, C07K 16/00, G01N 30/88, G01N 30/96, B01D 15/42, C07K 16/24, C07K 16/28, C07K 16/32

(54) **MASS SPECTROMETRY-COMPATIBLE PH GRADIENT BUFFER SYSTEM**
MASSENSPEKTROMETRIEKOMPATIBLES PH-GRADIENTENPUFFERSYSTEM
SYSTÈME DE TAMPON À GRADIENT DE PH COMPATIBLE AVEC LA SPECTROMÉTRIE DE MASSE

(30) Priority: 22.06.2021 US 202163213562 P; 30.03.2022 US 202263325392 P
(43) Date of publication of application: 01.05.2024
(73) Proprietor: Phenomenex, Inc., Torrance, CA 90501 (US)
(72) Inventor: SANCHEZ, Avelino, C., Torrance, CA 90505 (US)
(74) Representative: Simmons & Simmons
(86) International application number: PCT/US2022/034559
(87) International publication number: WO 2022/271853

(56) References cited:
- SCHAEFER W H ET AL: "Effect of high-performance liquid chromatography mobile phase components on sensitivity in negative atmospheric pressure chemical ionization liquid chromatography-mass spectrometry", JOURNAL OF THE AMERICAN SOCIETY FOR MASS SPECTROMETRY, ELSEVIER SCIENCE INC, US, vol. 7, no. 10, 1 October 1996 (1996-10-01), pages 1059 - 1069, XP004697692, ISSN: 1044-0305, DOI: 10.1016/1044-0305(96)00049-9
- FARSANG EVELIN ET AL: "Coupling non-denaturing chromatography to mass spectrometry for the characterization of monoclonal antibodies and related products", J. PHARM. BIOMED. ANAL., vol. 185, 25 February 2020 (2020-02-25), pages 1 - 14, XP055964873, Retrieved from the Internet <URL:https://www.sciencedirect.com/science/article/pii/S0731708519330742/pdfft?md5=45013d3a4e7eb21ef9790a2329e11e90&pid=1-s2.0-S0731708519330742-main.pdf> [retrieved on 20220926]
- FARSANG EVELIN ET AL: "Tuning selectivity in cation-exchange chromatography applied for monoclonal antibody separations, part 1: Alternative mobile phases and fine tuning of the separation", JOURNAL OF PHARMACEUTICAL AND BIOMEDICAL ANALYSIS, vol. 168, 19 February 2019 (2019-02-19), pages 138 - 147, XP085632722, ISSN: 0731-7085, DOI: 10.1016/J.JPBA.2019.02.024
- CHEN WEN-LING ET AL: "Ultra-high performance liquid chromatography/tandem mass spectrometry determination of feminizing chemicals in river water, sediment and tissue pretreated using disk-type solid-phase extraction and matrix solid-phase dispersion", TALANTA, vol. 89, 14 December 2011 (2011-12-14), pages 237 - 245, XP028888126, ISSN: 0039-9140, DOI: 10.1016/J.TALANTA.2011.12.020

## Description

### BACKGROUND

Monoclonal antibody (mAb) post-translational modifications (PTMs) are critical quality attributes of therapeutic mAbs. Post-translational modifications can occur during manufacturing, purification, and storage. PTMs can affect efficacy, activity, and stability of therapeutic mAbs. Primary amino acid sequence variations and PTMs may include C-terminal lysine truncation, N-terminal pyroglutamate formation, sialylation present on N-glycosylation, and deamidation of asparagine or glutamine residues. These modifications may vary the isoelectric point (pI) of the molecule resulting in acidic and basic charge variants of the mAb.

Monoclonal antibodies are routinely monitored during manufacturing and formulation for PTMs. Typical monitoring comprises use of ion-exchange chromatography (IEC) with ultraviolet monitoring (UV) using a salt or pH gradient for elution. Monitoring mAb PTMs with IEC coupled to native high-resolution mass spectrometry (HRMS) allows for rapid identification of modifications. Native MS of intact mAbs gives simpler spectra with less spectral overlap and less interference which facilitates identification of PTMs.

Improved mobile phase buffer compositions and methods for IEC-HRMS charge variant analysis of monoclonal antibodies demonstrating improved reproducibility, resolution, sensitivity, and pH linearity are desirable.

### SUMMARY OF THE INVENTION

The invention provides a ready-to-use mobile phase composition for liquid chromatography, according to claim 1. The composition may be used as a mobile liquid phase in liquid chromatography, according to claims 12 and 15. Further optional features are provided in the dependant claims.

The present disclosure thus provides pH gradient mobile phase buffer compositions and methods, giving good pH control across a wide range of pH values (pH 5.2-10.2) with low ionic strength (≤~50 mM, ≤~30 mM, or ≤~20mM) and with the advantages of giving improved MS sensitivity "more-native" mass spectra for intact proteins, giving lower charge states for protein isoforms, giving increased masses in the charge state envelope, and improved pH gradient linearity, robustness, and reproducibility.

Mobile phase pH gradient buffer compositions and methods are provided demonstrating improved chromatographic resolution of charge variants of intact monoclonal antibodies (mAbs), including low isoelectric point (pI) mAbs (e.g., Infliximab, pI 7.6), when using ion exchange chromatography.

The mobile phase buffer compositions and methods may be used in directly coupled IEC-HRMS of protein analytes, and demonstrate good MS sensitivity, for example, due to less than about 50 mM, less than 30 mM, less than 25 mM, or less than 20 mM salt concentration in the mobile phase buffer.

The mobile phase buffer compositions and methods may be used in IEC-HRMS of native protein analytes with improved mass spectrometric (MS) resolution due to lower charge states of intact proteins, i.e., MS spectra indicative of intact protein analytes with decreased denaturing.

The buffer compositions and methods provided herein are suitable for both UV and HRMS detection of intact protein analytes.

The disclosure provides a ready-to-use mobile phase composition for liquid chromatography comprising water; 2-50 mM of an ammonium carboxylate; 1-50 mM of an N-methylmorpholine, an isomer thereof, or an analog thereof and a pH in the range of about pH 4.5 to about pH 10.5 at room temperature. A ready-to-use mobile phase composition for liquid chromatography may comprise 2-25 mM of the ammonium carboxylate; and 1-10 mM of the N-methylmorpholine, the isomer thereof, or the analog thereof. The ammonium carboxylate may be ammonium acetate or ammonium formate. The N-methylmorpholine, the isomer thereof, or the analog thereof may be N-methylmorpholine. The N-methylmorpholine isomer may be selected from the group consisting of 2-methylmorpholine, 2-methylmorpholine, 2-methyl-1,3-oxazinane, 3-methyl-1,3-oxazinane, 4-methyl-1,3-oxazinane, 5-methyl-1,3-oxazinane, and 6-methyl-1,3-oxazinane. The N-methylmorpholine analog may be selected from the group consisting of an alkyl C₁-C₆ morpholine, a dialkyl C₁-C₆ morpholine, an alkyl C₁-C₆- 1,3-oxazinane, and a dialkylC₁-C₆- 1,3-oxazinane.

A ready-to-use mobile phase composition for liquid chromatography is provided comprising water; 2-25 mM ammonium acetate; 1-10 mM N-methylmorpholine; and a pH in the range of from about pH 4.5 to about pH 10.5 at room temperature.

The mobile phase may comprise a 2 part aqueous buffer system, wherein part A of the aqueous buffer system comprises from about 10 mM to about 20 mM ammonium acetate, about 3 to about 8 mM N-methylmorpholine, and a pH in the range of pH 5.0-5.5; and part B of the aqueous buffer system comprises 2-10 mM ammonium acetate, 1-5 mM N-methyl morpholine, and a pH in the range of pH 9.5 - 10.5. In some embodiments, the part A buffer may comprise from about 14 mM to about 16 mM of the ammonium acetate; about 4 mM to about 6 mM of the N-methylmorpholine; and a pH between about pH 5.1 and about pH 5.3. In some embodiments, the part B buffer may comprises from about 4 mM to about 6 mM of the ammonium acetate; about 1 mM to about 3 mM of the N-methylmorpholine; and a pH between about pH 10 and about pH 10.4.

The pH of the part A buffer may be adjusted with acetic acid. The pH of the part B buffer may be adjusted with ammonium hydroxide.

The ready-to-use mobile phase composition may comprise an ammonium acetate concentration of no more than 25 mM, no more than 20 mM, or no more than about 15 mM.

In some embodiments, the ready-to-use mobile phase composition may comprise no more than 100 ppb of an individual metal impurity.

The ready-to-use mobile phase composition may be used in a liquid chromatography method comprising a stationary phase. In some embodiments, the stationary phase may be selected from the group consisting of ion-exchange stationary phase, size-exclusion stationary phase, hydrophilic-interaction stationary phase, and reverse-phase stationary phase. In some embodiments, the ion-exchange stationary phase may be a cation exchange stationary phase.

The cation exchange stationary phase may be selected from the group consisting of a strong cation exchange stationary phase and a weak cation exchange stationary phase.

The ready-to-use mobile phase composition may be used in a method comprising liquid chromatography directly coupled (online) to a mass spectrometer.

The disclosure provides a method of separating and/or characterizing an analyte in a sample, comprising flowing a mobile phase through a chromatography column, wherein the mobile phase comprises a 2 part aqueous buffer system, wherein part A of the aqueous buffer system comprises from about 10-50 mM of an ammonium carboxylate, 3-16 mM of N-methylmorpholine, or isomer, or analog thereof, and a pH in a range between about pH 5 and about pH 5.5; and part B of the aqueous buffer system comprises 2-25 mM of an ammonium carboxylate, 1-10 mM of N-methyl morpholine, or isomer, or analog thereof, and a pH in a range between about pH 9.5 to about pH 10.5; injecting a sample comprising the analyte into the mobile phase; eluting the analyte from the column; and detecting the analyte in the eluate.

A method is provided for separating and/or characterizing an analyte in a sample, comprising flowing a mobile phase through a chromatography column, wherein the mobile phase comprises a 2 part aqueous buffer system, wherein part A of the aqueous buffer system comprises from about 10-25 mM ammonium acetate, 3-8 mM N-methylmorpholine, and a pH in a range between about pH 5 and about pH 5.5; and part B of the aqueous buffer system comprises 2-10 mM ammonium acetate, 1-5 mM N-methyl morpholine, and a pH in a range between about pH 9.5 to about pH 10.5; injecting a sample comprising the analyte into the mobile phase; eluting the analyte from the column; and detecting the analyte in the eluate.

The analyte may be a biomolecule. The biomolecule may be a monoclonal antibody, an antigen-binding fragment of a monoclonal antibody, or a charge variant thereof.

The monoclonal antibody or fragment may have a pI between about pI 6.5 and about pI 9.5.

The chromatography column may comprise a stationary phase selected from the group consisting of ion-exchange stationary phase, size-exclusion stationary phase, hydrophilic-interaction stationary phase, and reverse-phase stationary phase. The ion-exchange stationary phase may be a cation exchange stationary phase. The cation exchange stationary phase may be selected from the group consisting of a strong cation exchange stationary phase and a weak cation exchange stationary phase.

The detecting may comprise determining the UV absorbance of the eluate. The detecting may comprise detecting the analyte with a mass spectrometer (MS). The MS may be selected from the group consisting of sector, time-of-flight (TOF), quadrupole, ion trap, Fourier transform ion cyclotron resonance, and tandem (two or more of the above combined in tandem or orthogonal platforms) mass spectrometers.

The MS detection may comprise generating analyte ions. The generating may comprise an ionization technique selected from the group consisting of Electrospray ionization (ESI), Matrix-Assisted Laser Desorption/Ionization (MALDI), Fast Atom Bombardment (FAB), Chemical Ionization (CI), Electron Impact (EI), Atmospheric Solids Analysis Ionization (ASAI), Atmospheric Pressure Photoionization (APPI), Desorption Electrospray Ionization (DESI), and Atmospheric Pressure Vapor Source (APVS). The MS detection may further comprises acquiring a mass spectrum of the analyte ions. The MS detection may further comprise determining the molecular weight of the analyte.

In some embodiments, the mobile phase buffer compositions of the disclosure result in the mass spectra of the analyte ions exhibiting lower charge states for analyte isoforms than a comparable mobile phase buffer system comprising ammonium acetate without N-methylmorpholine. Use of the mobile phase buffer compositions of the disclosure may result in lower charge states for analyte isoforms and increased analyte masses in the charge state envelope than a comparable mobile phase buffer system comprising ammonium acetate without N-methylmorpholine.

In some embodiments, part A of the aqueous buffer system comprises from about 10-25 mM ammonium acetate, 3-8 mM N-methylmorpholine, and a pH in a range between about pH 5 and about pH 5.5; and part B of the aqueous buffer system comprises 2-10 mM ammonium acetate, 1-5 mM N-methyl morpholine, and a pH in a range between about pH 9.5 to about pH 10.5. The pH may be adjusted within a range of about pH 9.5 to about pH 10.5 with ammonium hydroxide. The mobile phase may contains no more than about 100 ppb of an individual metal impurity.

The elution may comprise forming a pH gradient. The pH gradient may comprise increasing the % buffer B over time relative to the % buffer A flowing through the column following the injection, wherein the % buffer A + % buffer B = 100% of the mobile phase. In some embodiments, the slope of the pH gradient is within a range of from 0.1-10%B/CV, or 0.5-5%B/column volume (CV) of the eluate. In some embodiments, the pH gradient may be a linear pH gradient, segmented pH gradient, curved pH gradient, or step pH gradient.

The disclosure provides a kit comprising a first container having a volume of a first concentrated liquid composition for dilution with water to obtain buffer A comprising 10-50 mM ammonium carboxylate, 3- 16 mM N-methylmorpholine, or an isomer, or an analog thereof, and a pH in a range between about pH 4.5 and about pH 5.5; a second container having a volume of a second concentrated liquid composition for dilution with water to obtain buffer B comprising 2-25 mM ammonium carboxylate, 1-10 mM N-methyl morpholine, or an isomer, or analog thereof, and a pH in a range between about pH 9.5 to about pH 10.5, and instructions for use.

A kit is provided comprising a first container having a volume of a first concentrated liquid composition for dilution with water to obtain buffer A comprising 10-25 mM ammonium acetate, 3- 8 mM N-methylmorpholine, and a pH in a range between about pH 4.5 and about pH 5.5; a second container having a volume of a second concentrated liquid composition for dilution with water to obtain buffer B comprising 2-10 mM ammonium acetate, 1-5 mM N-methyl morpholine, and a pH in a range between about pH 9.5 to about pH 10.5, and instructions for use. In some embodiments, the buffer A may comprise 10-20 mM ammonium acetate, 3- 8 mM N-methylmorpholine, and a pH in a range between about pH 5 and about pH 5.5. The buffer B may comprise buffer B comprising 2-10 mM ammonium acetate, 1-3 mM N-methyl morpholine, and a pH in a range between about pH 9.5 to about pH 10.5. The first and second concentrated liquid compositions may comprise a concentration that is selected from the group consisting of a 2X, 3X, 4X, 5X, 6X, 7X, 8X, 9X, 10 X, 11X, 12X, 13X, 14X, 15X, 16X, 17X, 18X, 19X, or 20X concentrate of the buffer A and the buffer B, respectively. The kit may further comprise a chromatography column. For example, the chromatography column may be a strong ion exchange chromatography column or a weak ion exchange chromatography column.

### BRIEF DESCRIPTION OF THE DRAWINGS

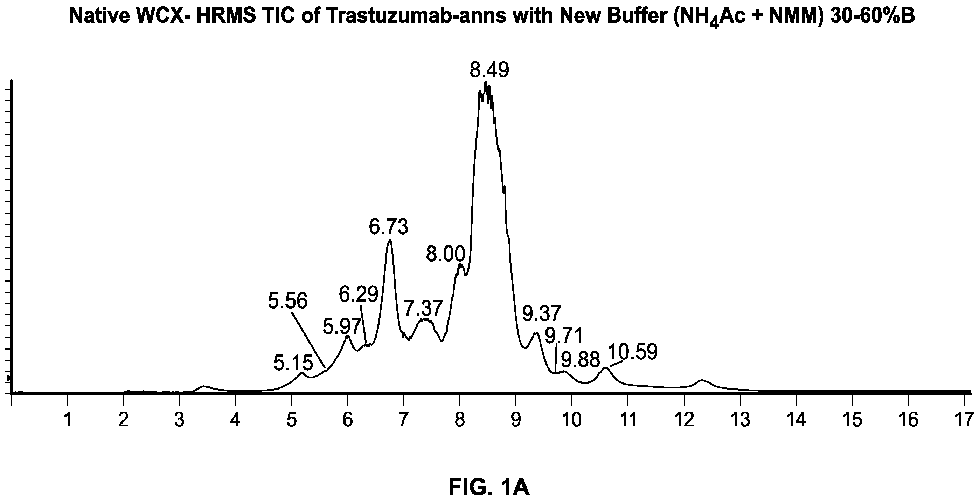

FIG. 1A shows a HRMS total ion current (TIC) chromatogram from Trastuzumab-anns on a Phenomenex^{®} bioZen WCX weak cation exchange column using new buffer system with NH₄Ac + NMM. Monoclonal antibody Trastuzumab-anns (Kajinti, 10 mg/mL) having pI 9.1 was subjected to native WCX-HRMS using the New Buffer; 10 uL (100 ug) was injected onto the Phenomenex^{®} bioZen 6µm WCX, 150 x 2.1 mm weak cation exchange column, using a 30-60%B gradient over 15 min according to Table 3, +TOF MS (2000-7000).

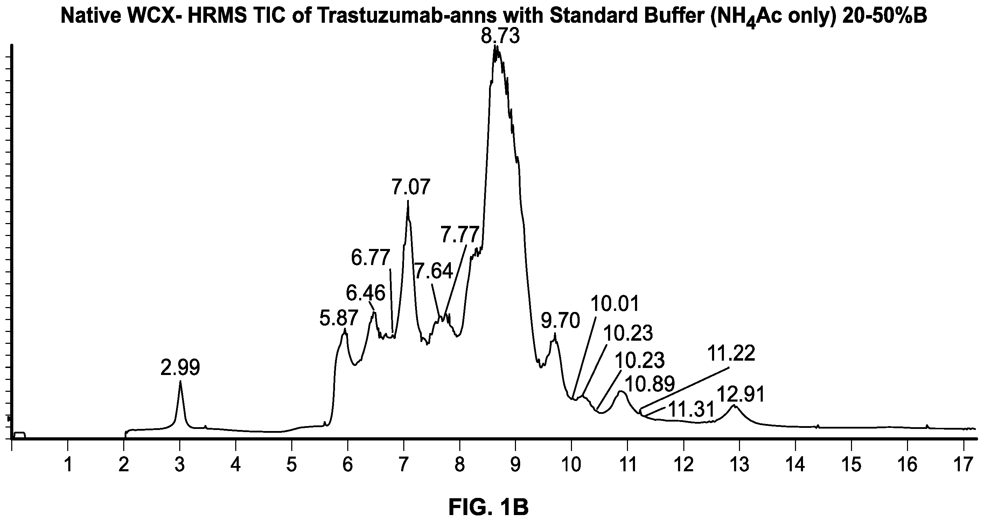

FIG. 1B shows a total ion current (TIC) chromatogram for Trastuzumab-anns on a Phenomenex^{®} bioZen WCX column using the standard buffer system NH₄Ac without NMM. Monoclonal antibody Trastuzumab-anns (Kajinti, 10 mg/mL) having pI 9.1 was subjected to native WCX-HRMS using the optimized standard buffer using a gradient of 20-50%B over 15 min according to Table 2; 10 uL (100 ug) was injected onto Phenomenex^{®} bioZen 6µm WCX, 150 x 2.1mm weak cation exchange column according to Table 2, +TOF MS (2000-7000).

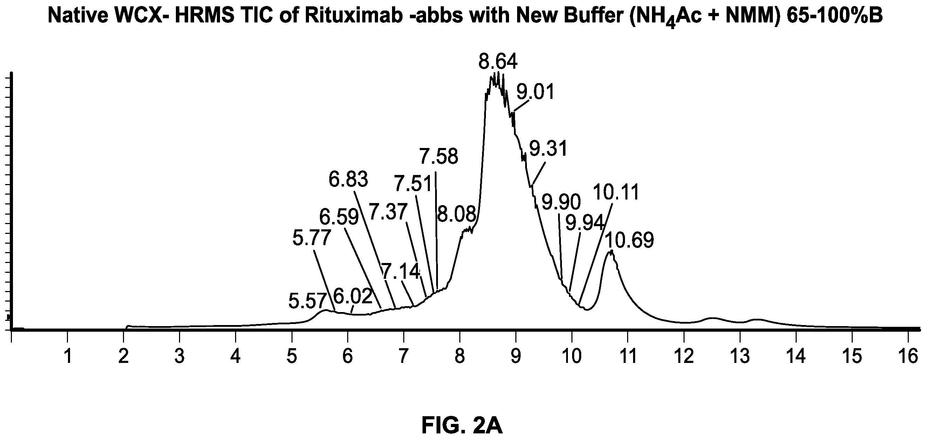

FIG. 2A shows a total ion current (TIC) chromatogram for Rituximab-abbs on a Phenomenex^{®} bioZen WCX column using the new buffer system with NH4Ac + NMM. Monoclonal antibody Rituximab-abbs (Rituxan) (10 mg/mL) having pI 9.4 was subjected to native WCX-HRMS using the New Buffer. A 10 uL (100 ug) aliquot was injected onto a Phenomenex^{®} bioZen 6µm WCX, 150 x 2.1mm weak cation exchange column and chromatographed using a 65-100%B gradient over 15 min as shown in Table 4, +TOF MS (2000-7000).

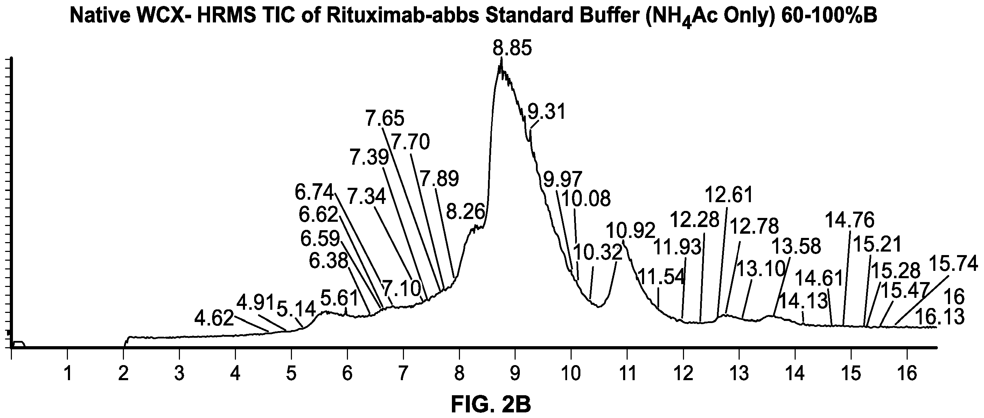

FIG. 2B shows a total ion current (TIC) chromatogram for Rituximab-abbs on a Phenomenex^{®} bioZen WCX column using the standard buffer system NH4Ac without NMM. Monoclonal antibody Rituximab-abbs (Rituxan, 10 mg/mL) having pI 9.4 was subjected to native WCX-HRMS using the optimized standard buffer with a 60-100%B gradient over 15 min as shown in Table 4; 10 uL (100 ug) was injected onto Phenomenex^{®} bioZen 6µm WCX, 150 x 2.1mm weak cation exchange column, +TOF MS (2000-7000).

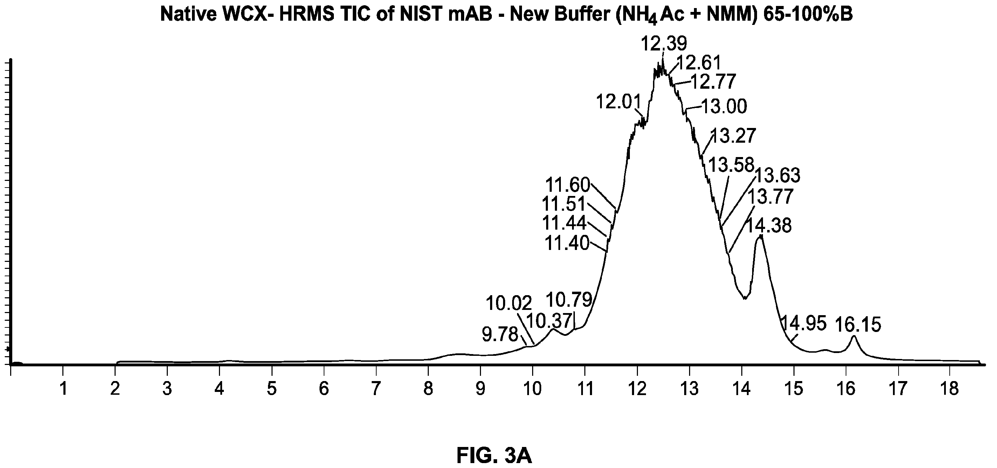

FIG. 3A shows a total ion current (TIC) chromatogram for NIST Reference mAb on a Phenomenex^{®} bioZen WCX column using the new buffer system with NH4Ac + NMM. Monoclonal antibody NIST Reference mAb (10 mg/mL) having pI 9.2 was subjected to native WCX-HRMS using the New Buffer; 10 uL (100 ug) was injected onto the bioZen 6µm WCX, 150 x 2.1mm weak cation exchange column, using a gradient of 65-100%B over 15 minutes, +TOF MS (2000-7000).

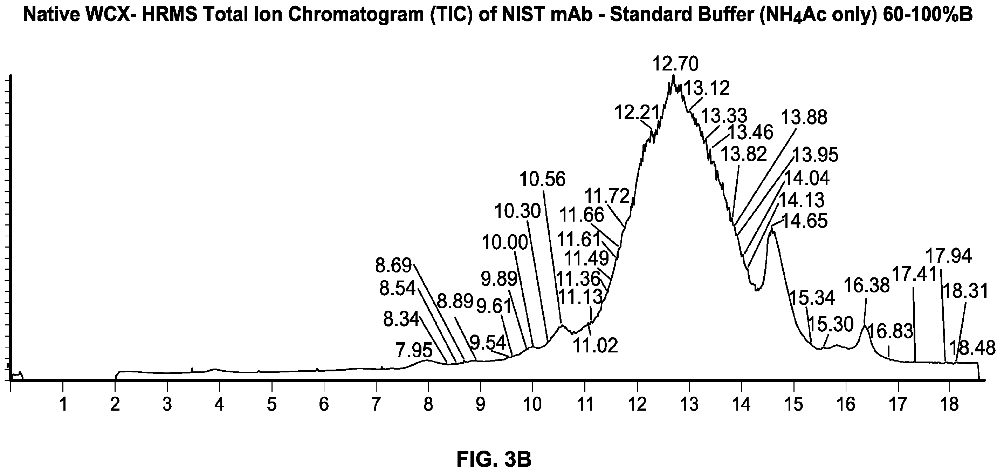

FIG. 3B shows a total ion current (TIC) chromatogram for NIST mAb on a Phenomenex^{®} bioZen WCX column using the standard buffer system NH4Ac without NMM. Monoclonal antibody NIST Reference mAb having pI 9.2 was subjected to native WCX-HRMS using the optimized standard buffer and 60-100%B gradient over 15 min as shown in Table 4; 10 uL (100 ug) was injected onto a Phenomenex^{®} bioZen 6µm WCX 150 x 2.1 mm weak cation exchange column, using the gradient shown in Table 4, +TOF MS (2000-7000).

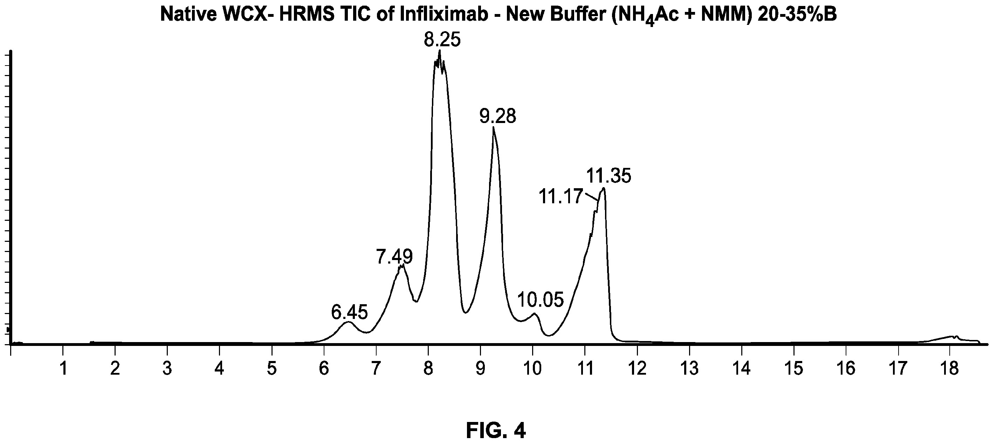

FIG. 4 shows a total ion current (TIC) chromatogram for Infliximab on a Phenomenex^{®} bioZen WCX column using the new buffer system with NH4Ac + NMM. Monoclonal antibody Infliximab (Remicade, 10 mg/mL) having pI 7.6 was subjected to native WCX-HRMS using the New Buffer and a gradient of 20-35%B over 15 min according to Table 5; 10 uL (100 ug) was injected onto the Phenomenex^{®} bioZen 6µm WCX 150 x 2.1 mm weak cation exchange column, +TOF MS (2000-7000).

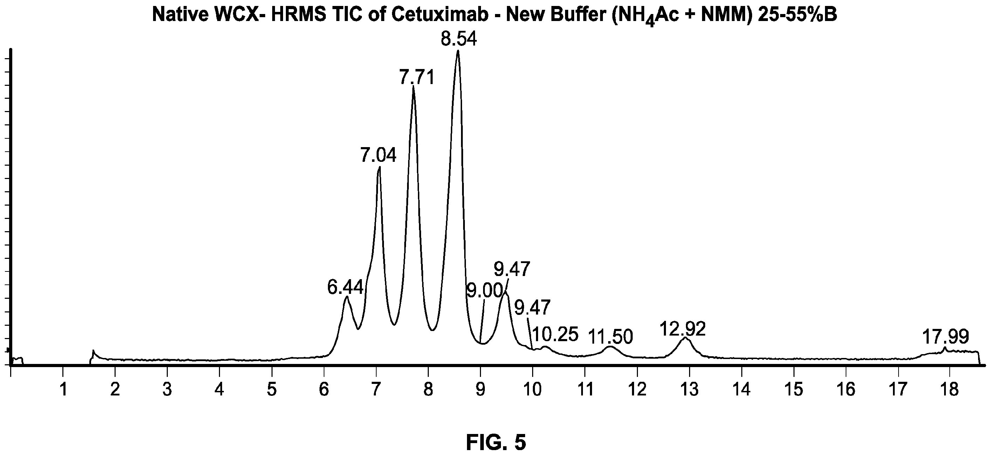

FIG. 5 shows a total ion current (TIC) chromatogram for Cetuximab on a Phenomenex^{®} bioZen WCX column using the new buffer system with NH4Ac + NMM. Monoclonal antibody Cetuximab (Erbitux; 10 mg/mL) having pI 8.8 was subjected to native WCX-HRMS using the New Buffer and a gradient of 25-55%B over 15 min according to Table 6; 10 uL (100 ug) was injected onto the Phenomenex^{®} bioZen 6µm WCX, 150 x 2.1 mm weak cation exchange column, +TOF MS (2000-7000).

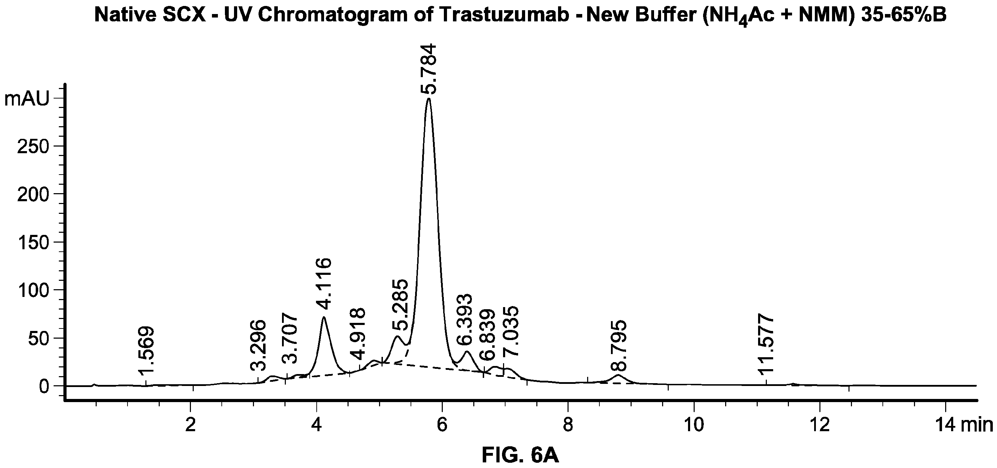

FIG. 6A shows a UV chromatogram monitored by Abs 280 nm of Trastuzumab-anns (Kanjinti) (10 mg/mL; 4 uL inj). The monoclonal antibody was injected onto a strong cation exchange column (experimental 3 µm SCX, 100 x 2.1mm) using the new buffer system containing NH4Ac + NMM and a pH gradient of 35-65% B over 10 min at flow rate of 0.3 mL/min.

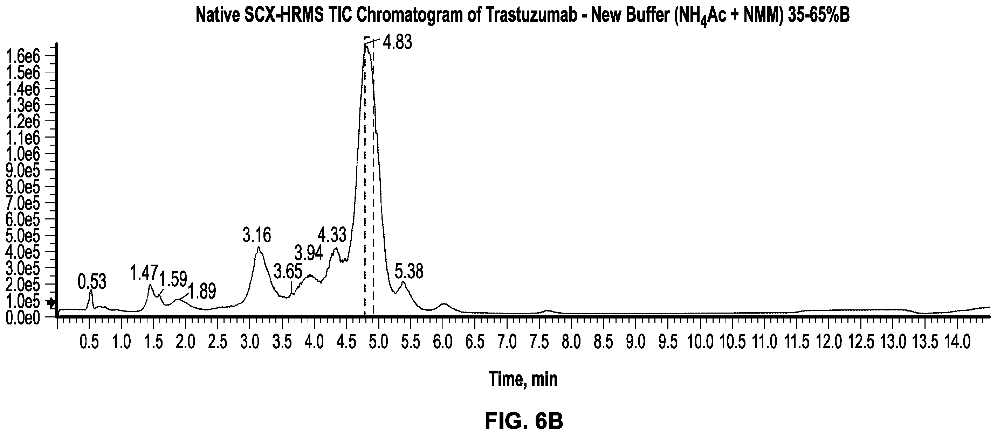

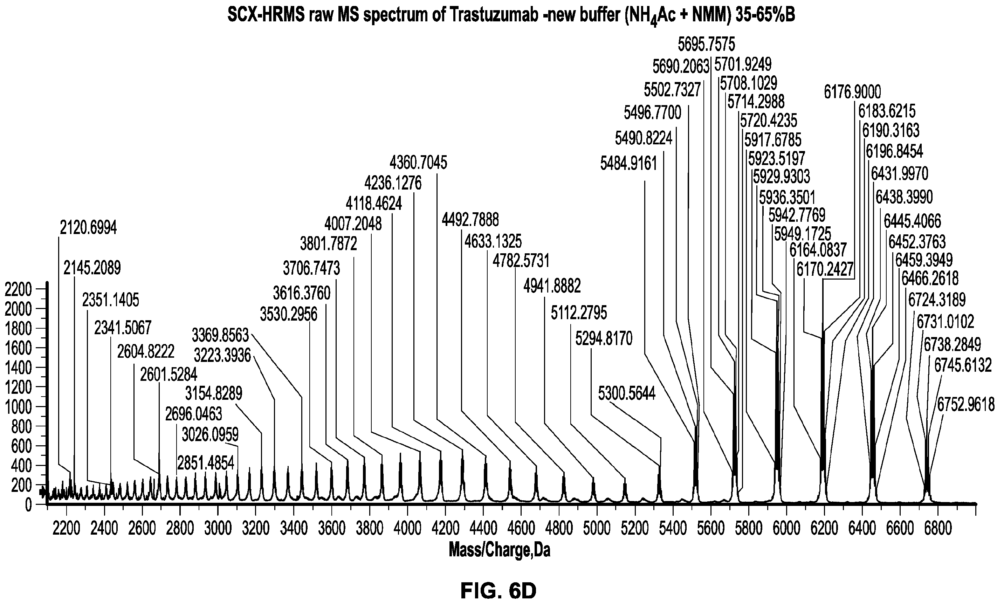

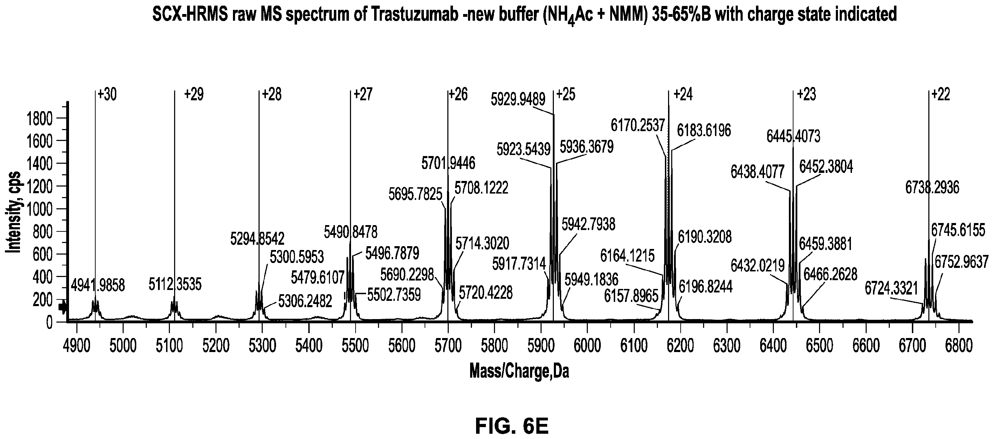

FIG. 6B shows a total ion current (TIC) chromatogram from the SCX-HRMS analysis of Trastuzumab-anns (4uL inj, 10 mg/mL) using the strong cation exchange LC column (experimental 3 µm SCX, 100 x 2.1mm) with the new buffer system containing NH4Ac + NMM and a pH gradient of 35-65% B over 10 min at flow rate of 0.3 mL/min, 30 °C; +TOF (2000-7000), MS temp 500 °C. The dotted line forming a box within the major peak at 4.83 min RT illustrates the approximate area for MS analysis as shown in FIG. 6D and FIG. 6E.

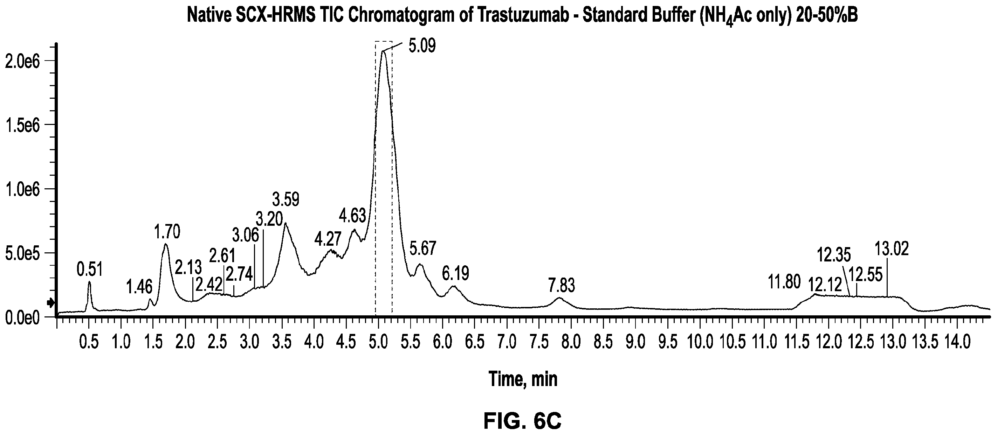

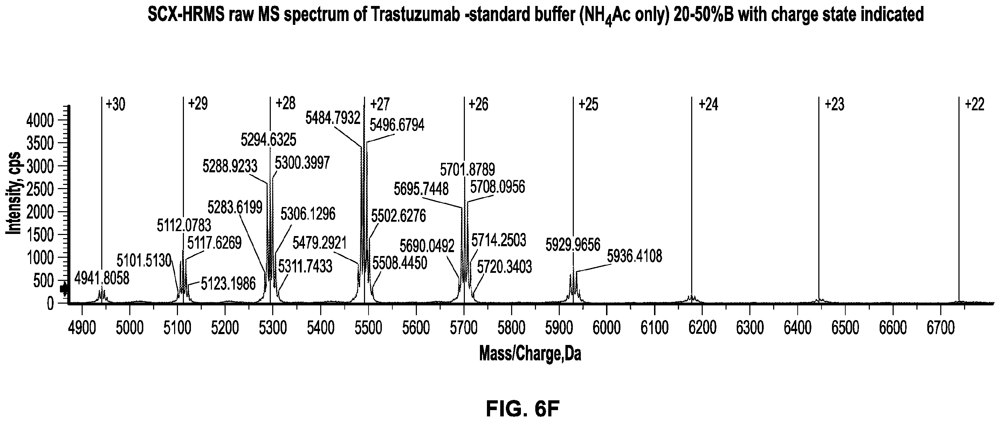

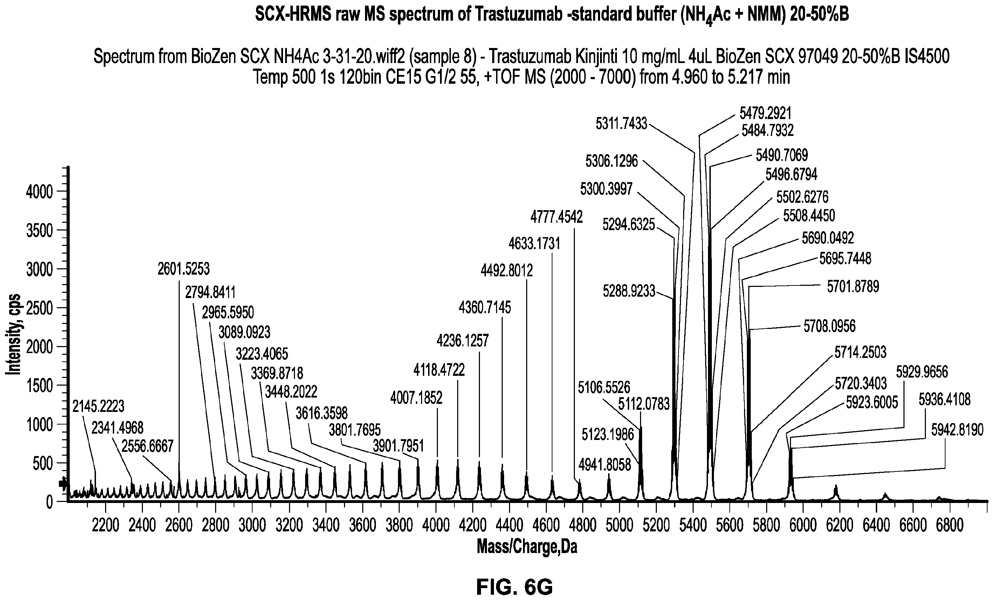

FIG. 6C shows a total ion current (TIC) chromatogram from SCX-HRMS of Trastuzumab-anns (4uL inj, 10 mg/mL) using the strong cation exchange LC column (experimental 3 µm SCX, 100 x 2.1mm) with the standard buffer system containing NH4Ac only and a pH gradient of 20-50% B over 10 min, 30 °C; +TOFMS (2000-7000), MS temp 500 °C. The dotted line forming a box within the major peak at 5.09 min RT illustrates the approximate area for MS analysis shown in FIG. 6F and FIG. 6G.
FIG. 6D shows raw MS spectrum from SCX-HRMS of Trastuzumab-anns (4ul inj, 10 mg/mL) using the strong cation exchange column (experimental 3 µm SCX, 100 x 2.1mm) with the new buffer system containing NH4Ac + NMM and a pH gradient of 35-65% B over10 min, +TOFMS (2000-7000) from 4.909 to 5.131 min. The mass/charge (m/z) range of 2000 to 7000 Da is shown.
FIG. 6E shows raw MS spectrum from SCX-HRMS of Trastuzumab-anns (4ul inj, 10 mg/mL) having pI 9.1, from 4.858 to 5.217 min, using the strong cation exchange LC column (experimental 3 µm SCX, 100 x 2.1mm) with new buffer system containing NH4Ac + NMM and a pH gradient of 35-65% B over10 min, +TOFMS (2000-7000). The m/z range displayed was narrowed to those giving the highest intensities for the native analyte (~4800-6600 Da) with this buffer system. Additionally, the charge of the analyte ions in the charge state envelope are indicated. With the new buffer system, the most intense MS peaks appear at higher masses/lower charges (+22 to +27) than seen with optimized standard NH4Ac buffer system (+25 to +30), as shown in FIG. 6F. The same mass range is used for both spectra, 6E and 6F.
FIG. 6F shows raw MS spectrum from SCX-HRMS of Trastuzumab-anns (4ul inj, 10 mg/mL) having pI 9.1, from 4.960 to 5.217 min, using strong cation exchange LC column (experimental 3 µm SCX, 100 x 2.1mm) with standard buffer system containing NH4Ac with no NMM and a pH gradient of 20-50% B/10 min, +TOFMS (2000-7000) . The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH4Ac + NMM buffer system (+22 to +27), as shown in FIG. 6E.
FIG. 6G shows raw MS spectrum from SCX-HRMS of Trastuzumab-anns (4ul, 10 mg/mL) having pI 9.1 using strong cation exchange column (experimental 3 µm SCX, 100 x 2.1mm) with optimized standard buffer system containing NH4Ac with no NMM and a pH gradient of 20-50% B/10 min, +TOF(2000-7000) from 4.960 to 5.217 min. Mass/charge range from 2000 to 7000 Da is shown.

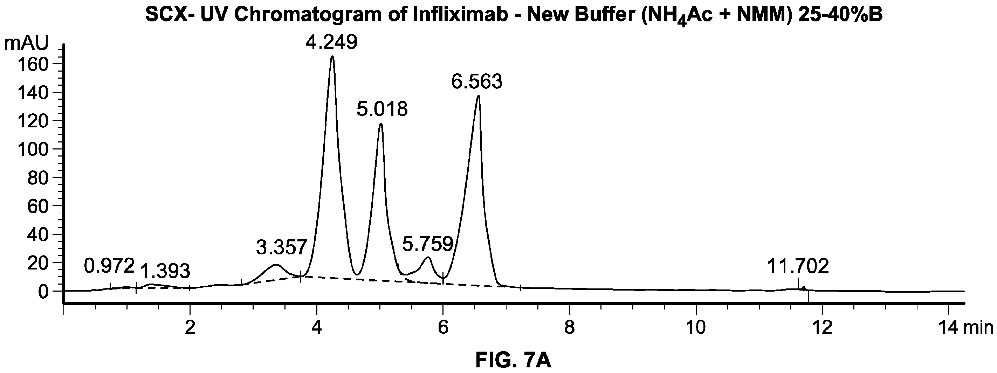

FIG. 7A shows a UV chromatogram monitored by Abs 280 nm from the SCX-UV analysis of Infliximab (10 mg/mL; 4 uL inj) using strong cation exchange column (experimental 3 µm SCX, 100 x 2.1mm) with new buffer system containing NH4Ac + NMM and a pH gradient of 25-40% B over10 min at flow rate of 0.3 mL/min. Good separation of variants was observed using a pH gradient with new buffer system: five distinct peaks were observed eluting at 3.357,4.249, 5.018, 5.759, and 6.563 min RT.

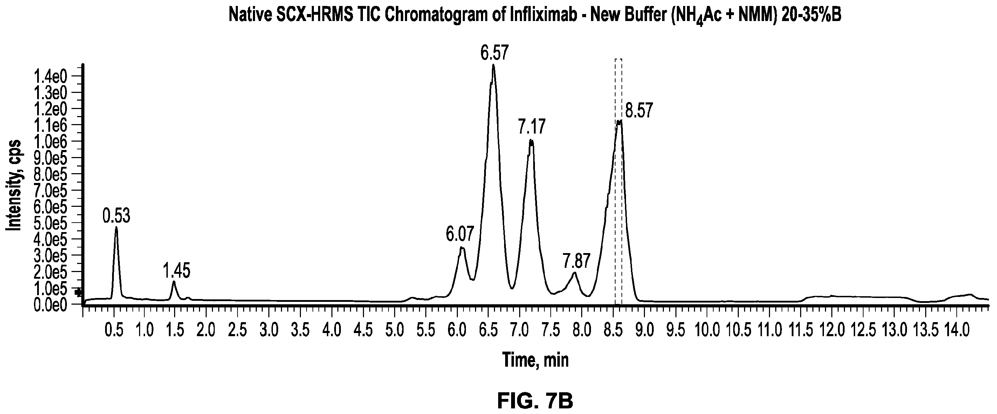

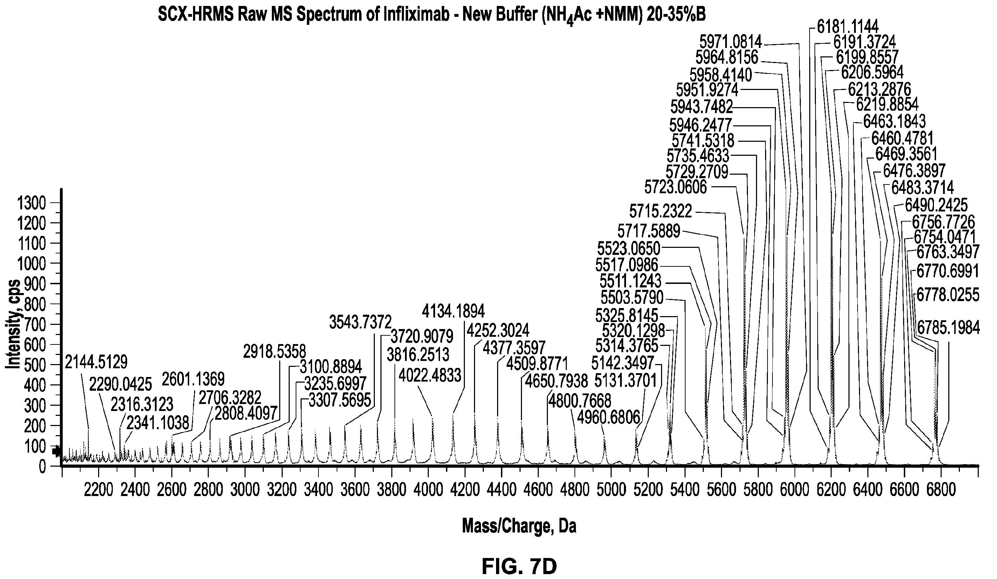

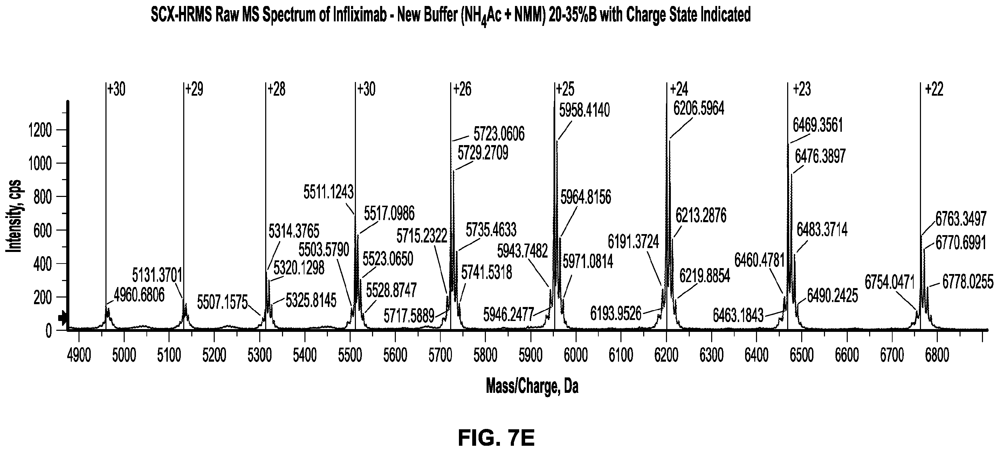

FIG. 7B shows a total ion current (TIC) chromatogram from SCX-HRMS of Infliximab (4ul, 5 mg/mL) using a strong cation exchange LC column (experimental 3 µm SCX, 100 x 2.1mm) with new buffer system containing NH4Ac + NMM and a pH gradient of 20-35% B over 10 min at flow rate of 0.3 mL/min, 30 °C; +TOF(2000-7000), MS temp 500 °C. Good separation of variants was observed using pH gradient with new buffer system: five distinct peaks were observed eluting at 6.07, 6.57, 7.17, 7.87, and 8.57 min RT. The dotted line forming a box within the major peak at 8.57 min RT illustrates the approximate area for MS analysis as shown in FIG. 7D and FIG. 7E.

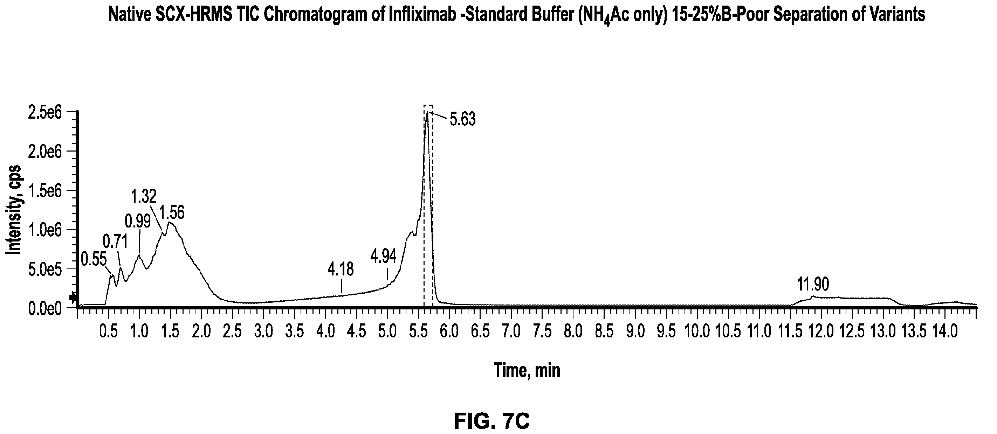

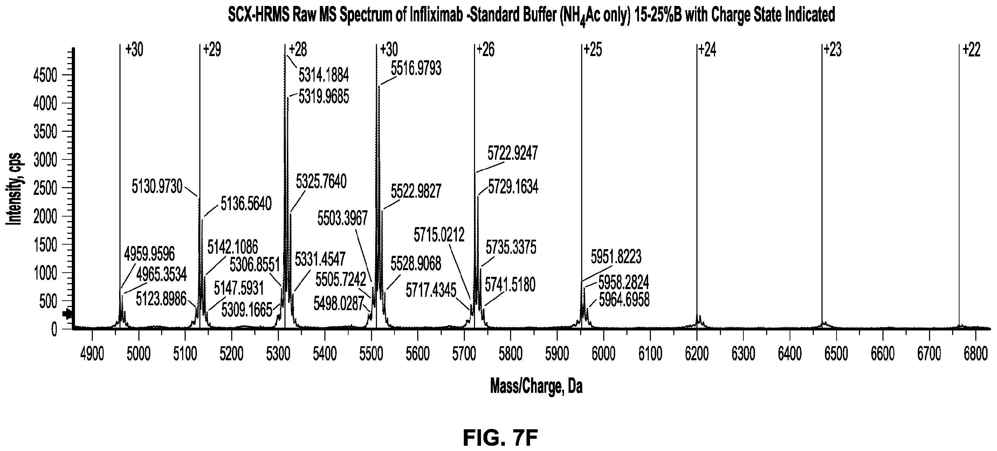

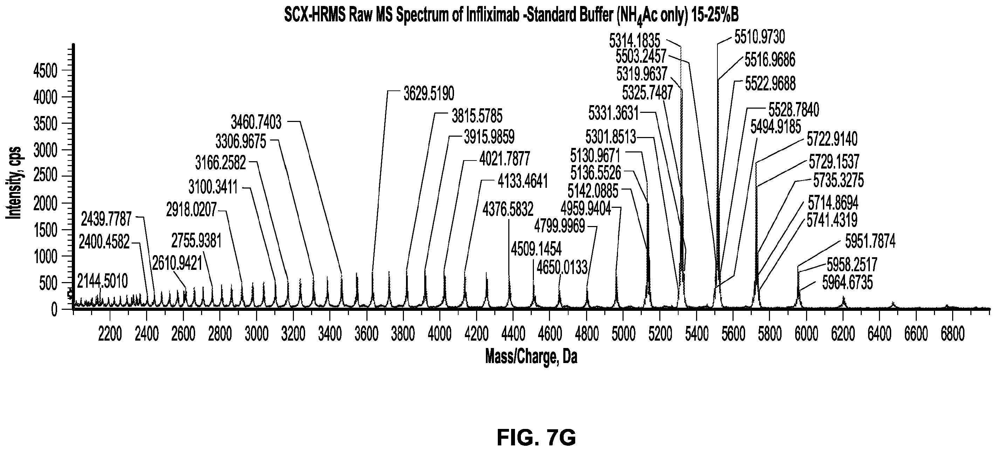

FIG. 7C shows a total ion current (TIC) chromatogram from SCX-HRMS of Infliximab (4ul, 5 mg/mL) using a strong cation exchange LC column (experimental 3 µm SCX, 100 x 2.1mm) with standard buffer system containing NH4Ac only and a pH gradient of 15-25% B over 10 min, 30 °C; +TOF(2000-7000), MS temp 500 °C. The dotted line forming a box within the major peak at 5.63 min RT illustrates the approximate area for MS analysis shown in FIG. 7F and FIG. 7G. Very poor separation of charge variants was given with the standard buffer for this low pI mAb (pI = 7.6). Comparison of this figure with the previous one (Figure 7B) demonstrates the performance benefit obtained by having good pH control at lower pHs than those routinely possible with the standard buffer.
FIG. 7D shows raw MS spectrum from SCX-HRMS of Infliximab (4ul, 5 mg/mL) using a strong cation exchange column (experimental 3 µm SCX, 100 x 2.1mm) with new buffer system containing NH4Ac + NMM and a pH gradient of 20-35% B/10 min, +TOF(2000-7000) from 8.621 to 9.032 min. Mass/charge range from 2000 to 7000 Da is shown.
FIG. 7E shows raw MS spectrum from SCX-HRMS of Infliximab (4ul, 5 mg/mL) from 8.621 to 9.032 min, using strong cation exchange LC column (experimental 3 µm SCX, 100 x 2.1mm) with new buffer system containing NH4Ac + NMM and a pH gradient of 20-35% B over 10 min, +TOF(2000-7000) The m/z range displayed was narrowed to that giving the highest intensities for the native analyte (~4800-6900 Da) with this buffer system. Additionally, the charge of the analyte ions in the charge state envelope are indicated. As shown in FIG. 7E, with the new buffer system, the most intense MS peaks appear at higher masses/lower charges (+22 to +27) than seen with optimized standard NH4Ac buffer system (+25 to +30), shown in FIG. 7F.
FIG. 7F shows raw MS spectrum from SCX-HRMS of Infliximab (4ul, 5 mg/mL), from 5.593 to 5.730 min, using strong cation exchange LC column (experimental 3 µm SCX, 100 x 2.1mm) with standard buffer system containing NH4Ac with no NMM and a pH gradient of 15-25% B over 10 min, +TOF(2000-7000). The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH4Ac + NMM buffer system (+22 to +27), as shown in FIG. 7E.
FIG. 7G shows raw MS spectrum from SCX-HRMS of Infliximab (4ul, 5 mg/mL), using strong cation exchange column (experimental 3 µm SCX, 100 x 2.1mm) with optimized standard buffer system containing NH4Ac with no NMM and a pH gradient of 15-25% B/10 min, +TOF(2000-7000) from 5.559 to 5.696 min. Mass/charge range from 2000 to 6900 Da is shown.

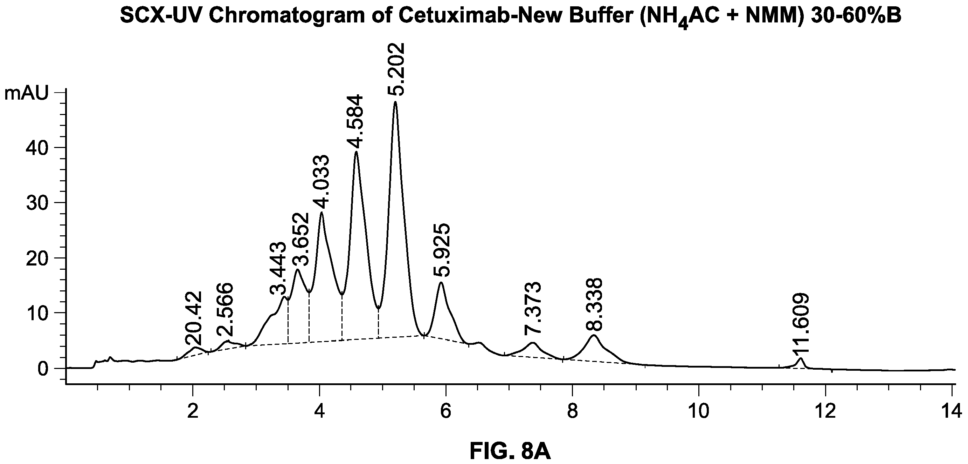

FIG. 8A shows a UV chromatogram by Abs 280 nm from SCX-UV analysis of Cetuximab (4 mg/mL) using a strong cation exchange column (experimental 3 µm SCX, 100 x 2.1mm) with new buffer system containing NH4Ac + NMM and a pH gradient of 30-60% B over 10 min at flow rate of 0.3 mL/min. Multiple distinct variant peaks were observed, e.g., at 3.65, 4.03, 4.58, 5.20, and 5.93 min RT.

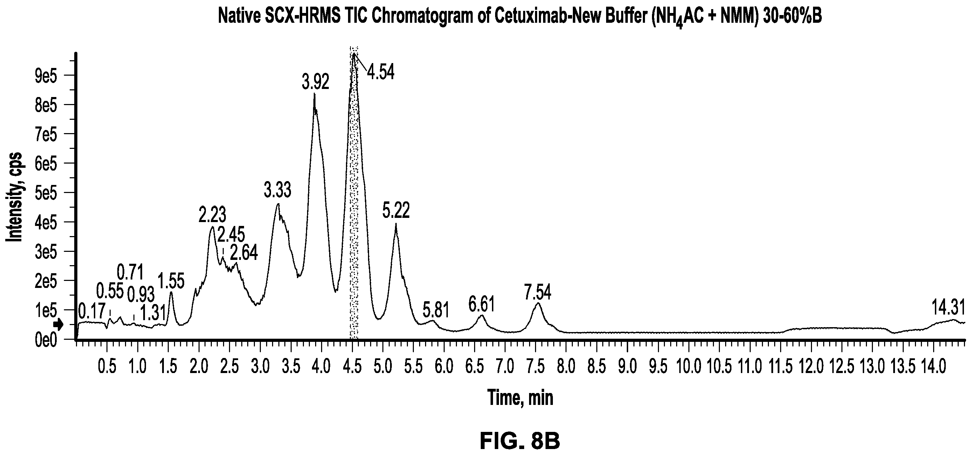

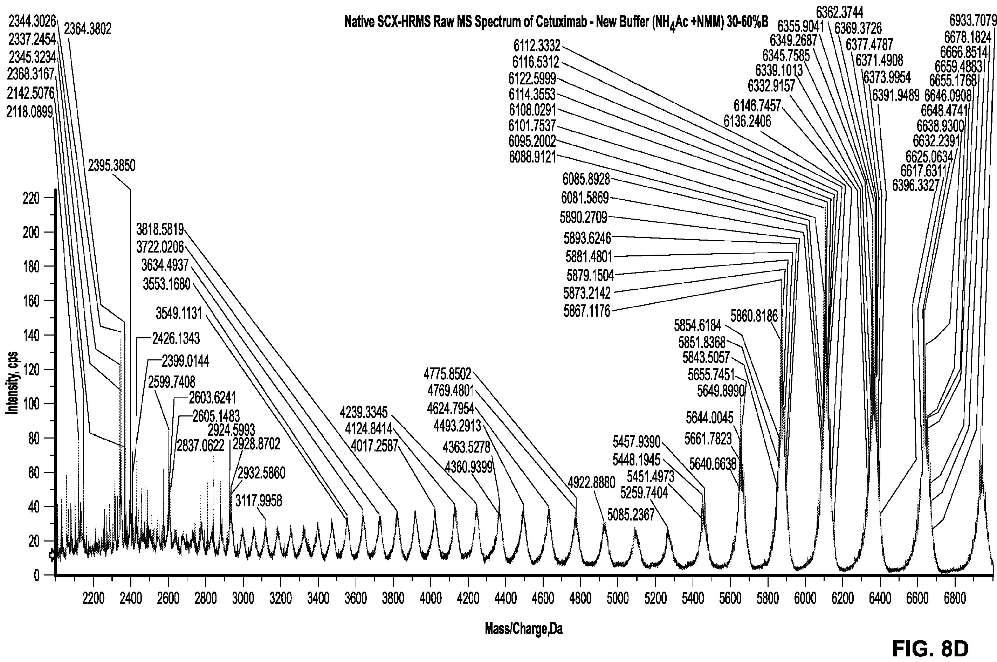

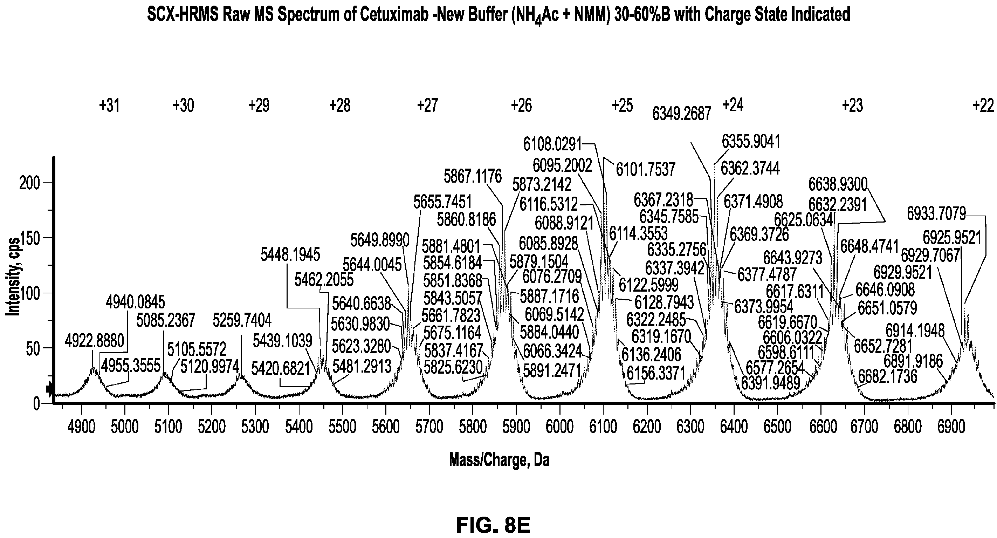

FIG. 8B shows a total ion current (TIC) chromatogram from SCX-HRMS of Cetuximab (5 mg/mL; 10 uL inj) using a strong cation exchange LC column (experimental 3 µm SCX, 100 x 2.1mm) with the new buffer system containing NH4Ac + NMM and a pH gradient of 30-60% B over10 min at flow rate of 0.3 mL/min, 30 °C; +TOF(2000-7000), MS temp 500 °C. Multiple distinct variant peaks were observed at 3.33, 3.92, 4.54, and 5.22 min RT. The dotted line forming a box within the major peak at 4.54 min RT illustrates the approximate area for MS analysis as shown in FIG. 8D and FIG. 8E.

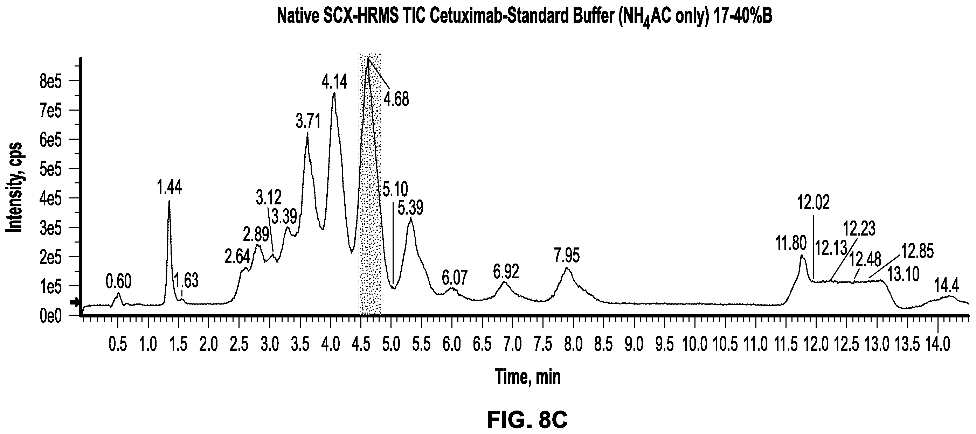

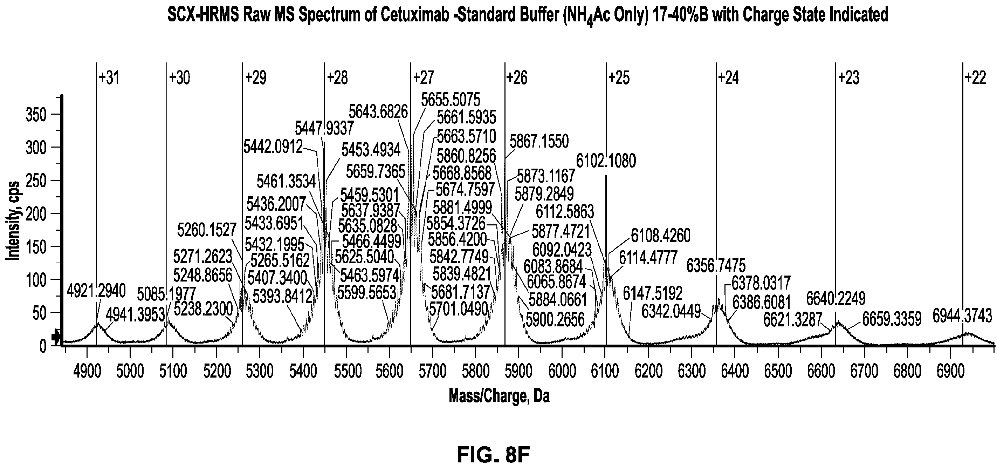

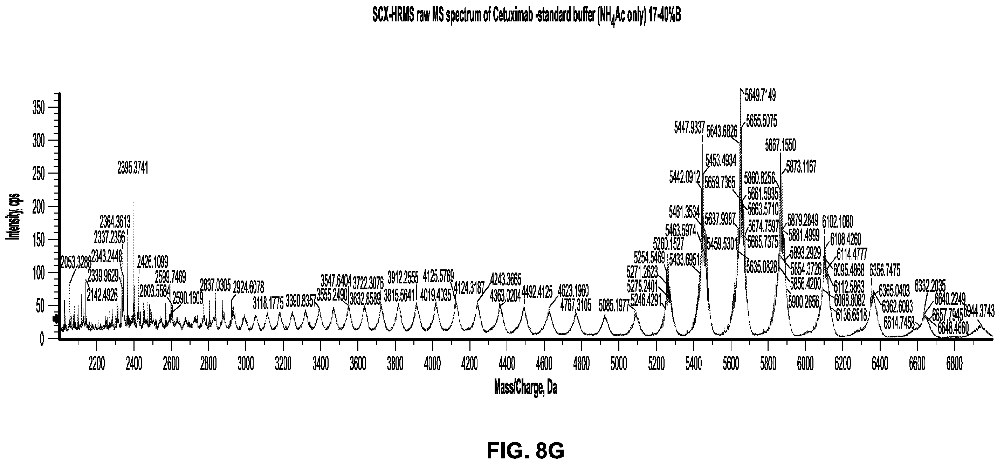

FIG. 8C shows a total ion current (TIC) chromatogram from the SCX-HRMS analysis of Cetuximab (5 mg/mL; 10 uL inj) using a strong cation exchange LC column (experimental 3 µm SCX, 100 x 2.1mm) with standard buffer system containing NH4Ac only and a pH gradient of 17-40% B/10 min, 30 °C; +TOF(2000-7000), MS temp 500 °C. The dotted line forming a box within the major peak at 4.68 min RT illustrates the approximate area for MS analysis shown in FIG. 8F and FIG. 8G.
FIG. 8D shows a raw MS spectrum from SCX-HRMS of Cetuximab (5 mg/mL; 10 uL inj) using a strong cation exchange column (experimental 3 µm SCX, 100 x 2.1mm) with new buffer system containing NH4Ac + NMM and a pH gradient of 30-60% B over 10 min, +TOF(2000-7000) from 4.601 to 9.032 min. Mass/charge range from 2000 to 7000 Da is shown.
FIG. 8E shows a raw MS spectrum from SCX-HRMS of Cetuximab (5 mg/mL; 10 uL inj) from 4.601 to 4.892 min, using a strong cation exchange LC column (experimental 3 µm SCX, 100 x 2.1mm) with new buffer system containing NH4Ac + NMM and a pH gradient of 30-60% B over 10 min, +TOF(2000-7000). The m/z range displayed was narrowed to that giving the highest intensities for the native analyte (~4800-6900 Da) with this buffer system. Additionally, the charge of the analyte ions in the charge state envelope are indicated. As shown in FIG. 8E, when using pH gradient elution with the new buffer system, the most intense MS peaks appear at higher masses/lower charges (+22 to +27) than seen with optimized standard NH4Ac buffer system (+25 to +30), as shown in FIG. 8F.
FIG. 8F shows raw MS spectrum from SCX-HRMS of Cetuximab (5 mg/mL; 10 uL inj), from 4.533 to 4.892 min, using a strong cation exchange LC column (experimental 3 µm SCX, 100 x 2.1mm) with the standard buffer system containing NH4Ac with no NMM and a pH gradient of 17-40% B over10 min,+TOF(2000-7000). The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH4Ac + NMM buffer system (+22 to +27), as shown in FIG. 8E.
FIG. 8G shows a raw MS spectrum from SCX-HRMS of Cetuximab (5 mg/mL; 10 uL inj), from 4.533 to 4.892 min. using a strong cation exchange column (experimental 3 µm SCX, 100 x 2.1mm) with the optimized standard buffer system containing NH4Ac with no NMM and a pH gradient of 17-40% B over 10 min, +TOF(2000-7000). Mass/charge range from 2000 to 6900 Da is shown.

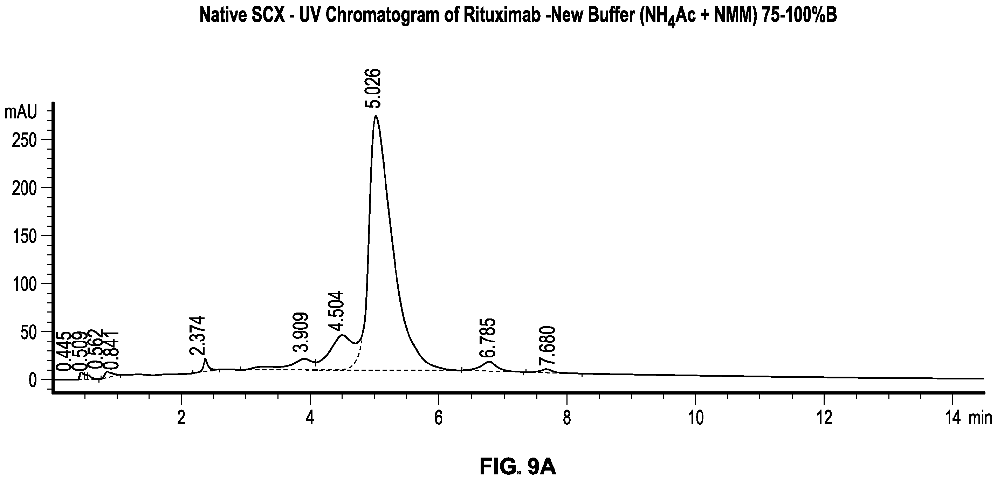

FIG. 9A shows a UV chromatogram at Abs 280 nm from SCX-HRMS of Rituximab (Rituxan) having pI 9.4 (10 mg/mL) using strong cation exchange column (experimental 3 µm SCX, 100 x 2.1mm) with the new buffer system containing NH4Ac + NMM and a pH gradient of 75-100% B over 10 min at flow rate of 0.3 mL/min. A major peak at 5.03 min RT was observed.

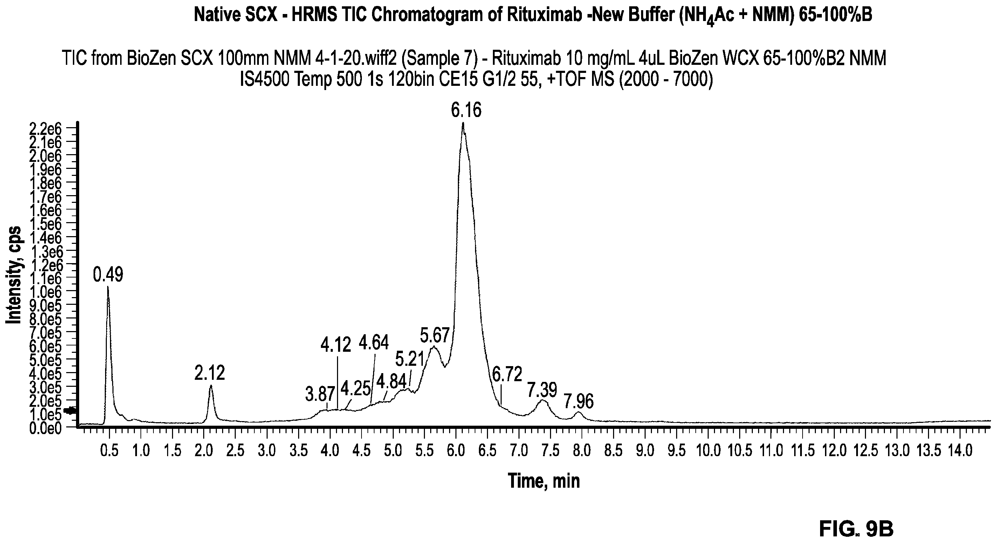

FIG. 9B shows a total ion current (TIC) chromatogram from the SCX-HRMS analysis of Rituximab (10 mg/mL; 4 uL inj) using strong cation exchange LC column (experimental 3 µm SCX, 100 x 2.1mm) with new buffer system containing NH4Ac + NMM and a pH gradient of 65-100% B over 10 min at flow rate of 0.3 mL/min, 30 °C; +TOFMS (2000-7000), MS temp 500 °C. The dotted line forming a box within the major peak at 5.88 min RT illustrates an approximate area for MS analysis.

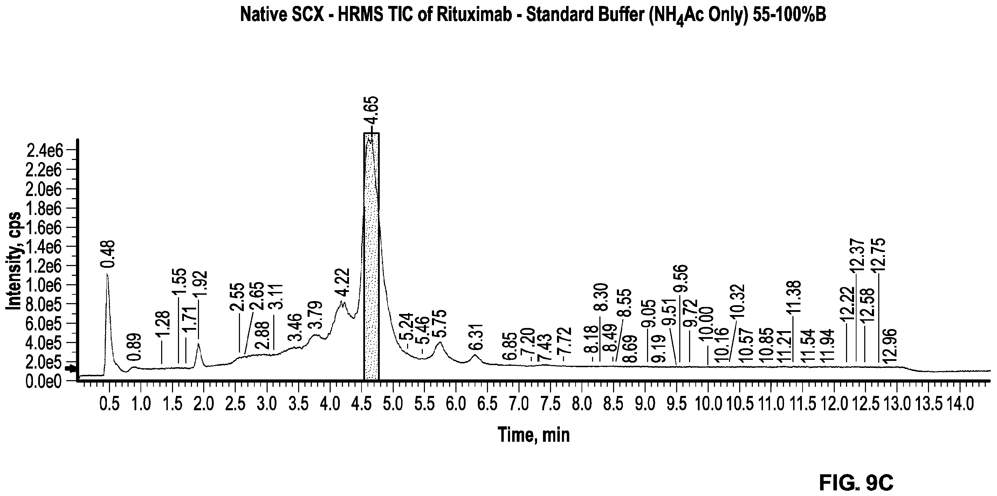

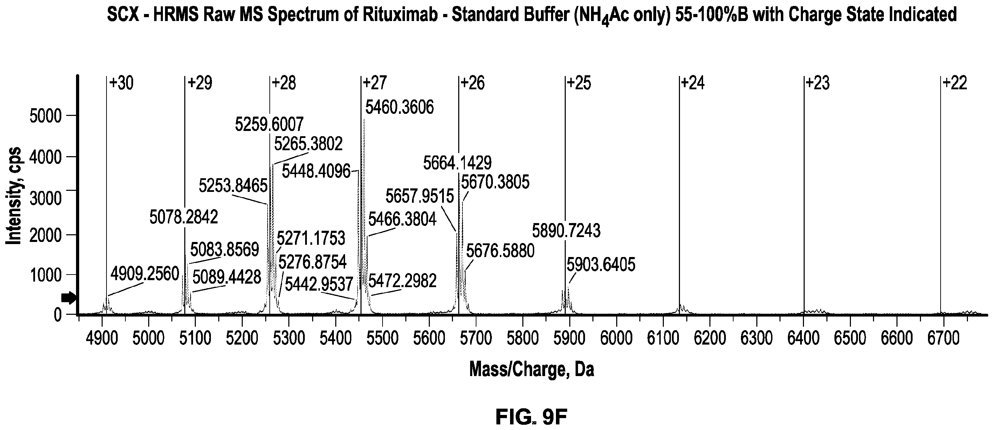

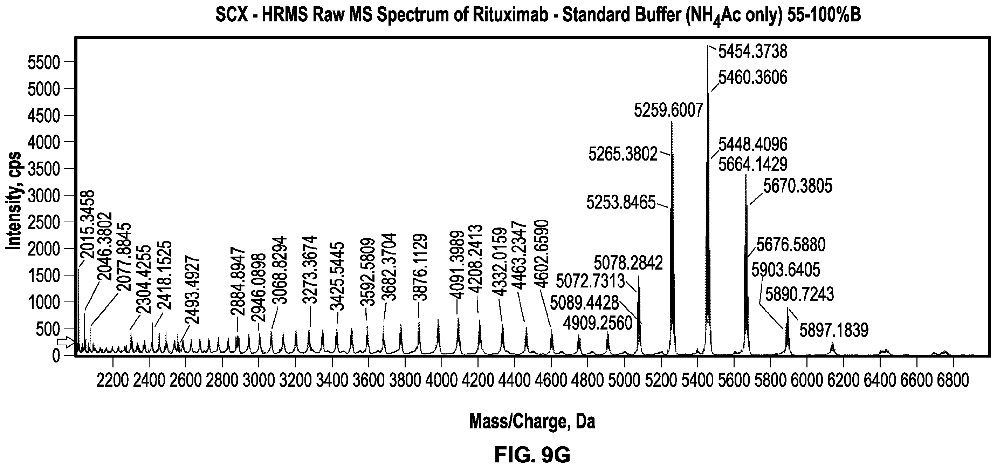

FIG. 9C shows a total ion current (TIC) chromatogram from the SCX-HRMS analysis of Rituximab (10 mg/mL; 4 uL inj) using a strong cation exchange LC column (experimental 3 µm SCX, 100 x 2.1mm) with standard buffer system containing NH4Ac only and a pH gradient of 55-100% B over 10 min, 30 °C; +TOFMS (2000-7000), MS temp 500 °C. The dotted line forming a box within the major peak at 4.65 min RT illustrates the approximate area for MS analysis shown in FIG. 9F and FIG. 9G.

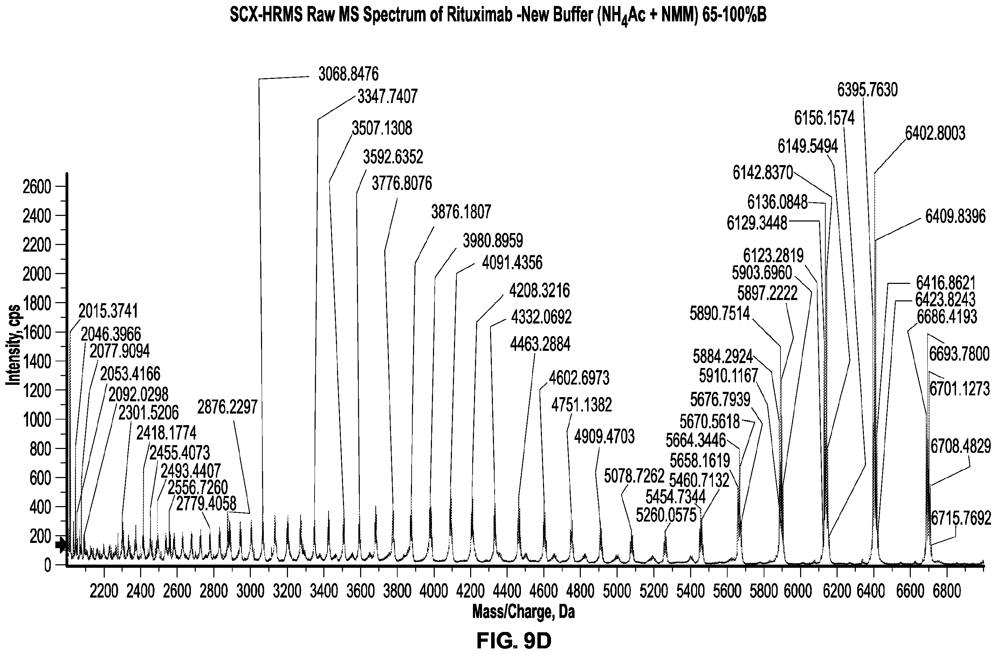

FIG. 9D shows a raw MS spectrum from LC-HRMS of Rituximab (10 mg/mL; 4 uL inj) using a strong cation exchange column (experimental 3 µm SCX, 100 x 2.1mm) with new buffer system containing NH4Ac + NMM and a pH gradient of 65-100% B over 10 min, +TOFMS (2000-7000) from 6.021 to 6.363 min. Mass/charge, m/z data in a range from 2000 to 7000 Da is shown.

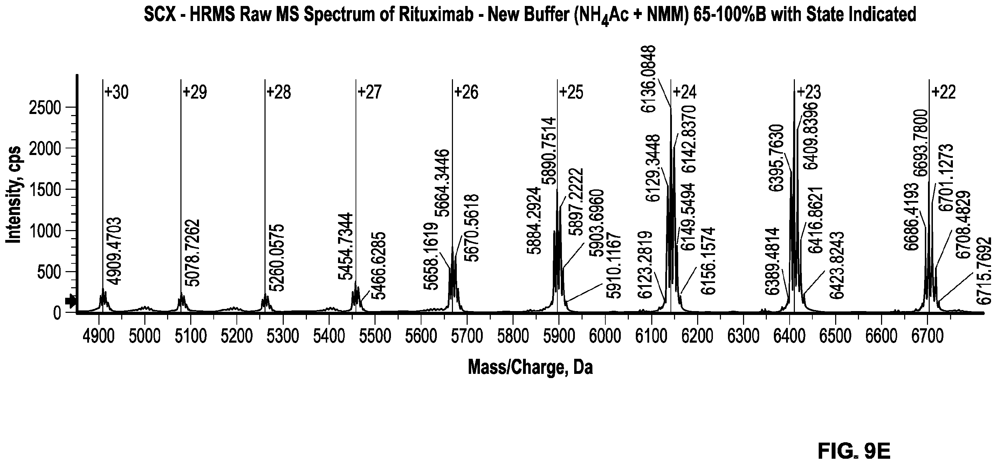

FIG. 9E shows a raw MS spectrum from the SCX-HRMS analysis of Rituximab (10 mg/mL; 4 uL inj) using a strong cation exchange LC column (experimental 3 µm SCX, 100 x 2.1mm) with new buffer system containing NH4Ac + NMM and a pH gradient of 65-100% B/10 min, +TOFMS (2000-7000), from 6.021 to 6.363 min. The m/z range displayed was narrowed to that giving the highest intensities for the native analyte (~4800-6800 Da) with this buffer system. Additionally, the charge of the analyte ions in the charge state envelope are indicated. As show in FIG. 9E, when using a pH gradient with new buffer system, the most intense MS peaks appear at higher masses/lower charges (+22 to +27) than seen with optimized standard NH4Ac buffer system (+25 to +30), shown in FIG. 9F.
FIG. 9F shows a raw MS spectrum from the SCX-HRMS analysis of Rituximab (10 mg/mL; 4 uL inj) using a strong cation exchange LC column (experimental 3 µm SCX, 100 x 2.1mm) with standard buffer system containing NH4Ac with no NMM and a pH gradient of 55-100% B over 10 min, +TOFMS (2000-7000), from 4.550 to 4.772 min. The m/z range displayed was narrowed to that giving the highest intensities for the native analyte (~4800-6800 Da) with this buffer system. Additionally, the charge of the analyte ions in the charge state envelope are indicated. With the optimized standard buffer system, the most intense MS peaks appear at lower masses/higher charges (+25 to +30), when compared to new NH4Ac + NMM buffer system (+22 to +27), as shown in FIG. 9E.
FIG. 9G shows a raw MS spectrum from the SCX-HRMS analysis of Rituximab (10 mg/mL; 4 uL inj) from 4.550 to 4.772 min. using a strong cation exchange column (experimental 3 µm SCX, 100 x 2.1mm) with the optimized standard buffer system containing NH4Ac with no NMM and a pH gradient of 55-100% B/10 min, +TOFMS (2000-7000). Mass/charge range from 2000 to 7000 Da is shown.

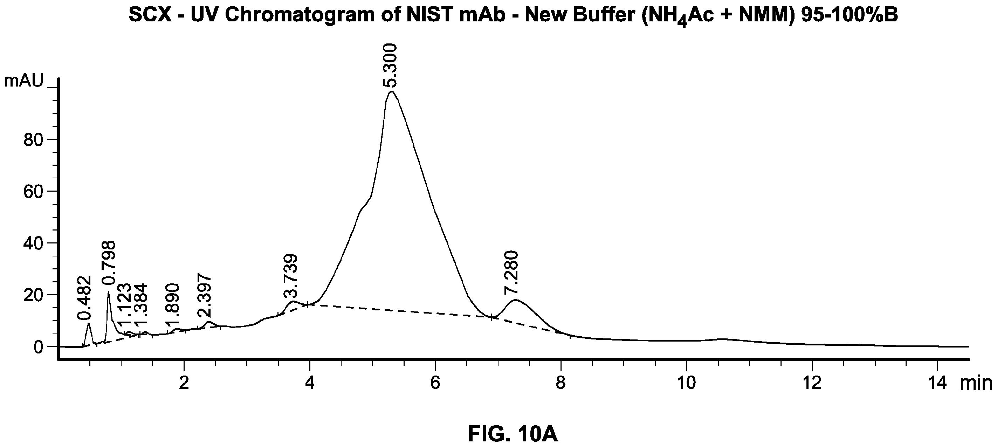

FIG. 10A shows a UV chromatogram monitored at Abs 280 nm from the SCX-UV analysis of NIST mAb having pI 9.2 (10 mg/mL) using a strong cation exchange column (experimental 3 µm SCX, 100 x 2.1mm) with the new buffer system containing NH4Ac + NMM and a pH gradient of 95-100% B over 10 min at flow rate of 0.3 mL/min.

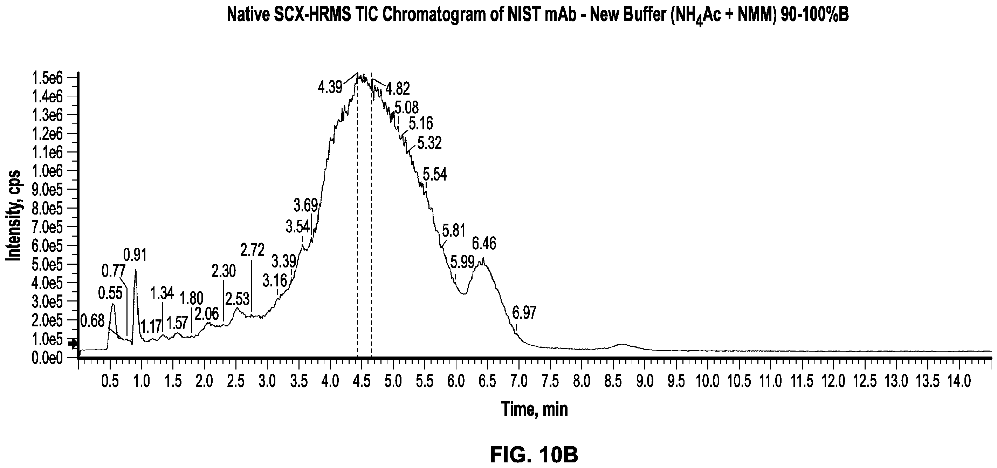

FIG. 10B shows a total ion current (TIC) chromatogram from SCX-HRMS of NIST mAb (10 mg/mL; 10 uL inj) using a strong cation exchange LC column (experimental 3 µm SCX, 100 x 2.1mm) with the new buffer system containing NH4Ac + NMM and a pH gradient of 90-100% B over 10 min at flow rate of 0.3 mL/min, 30 °C; +TOFMS (2000-7000), MS temp 500 °C. The dotted line forming a box within peak between 4.39 and 4.82 RT illustrates an approximate area for MS analysis.

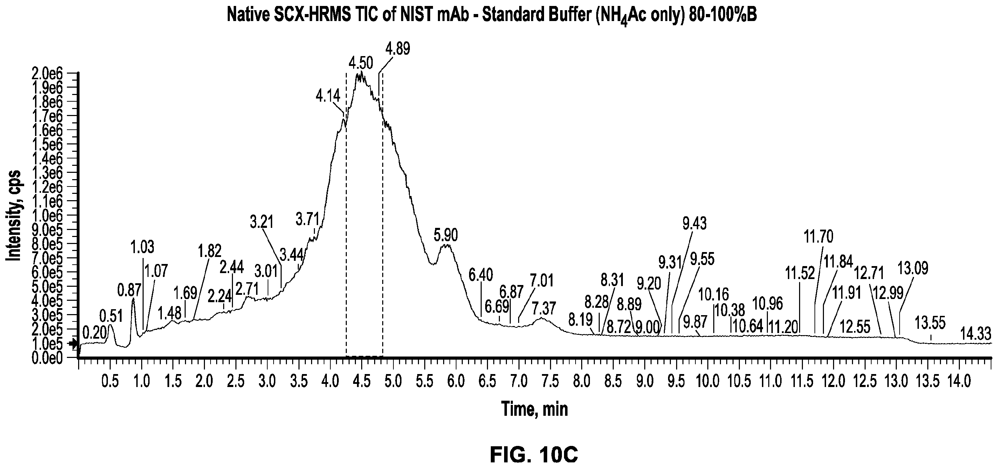

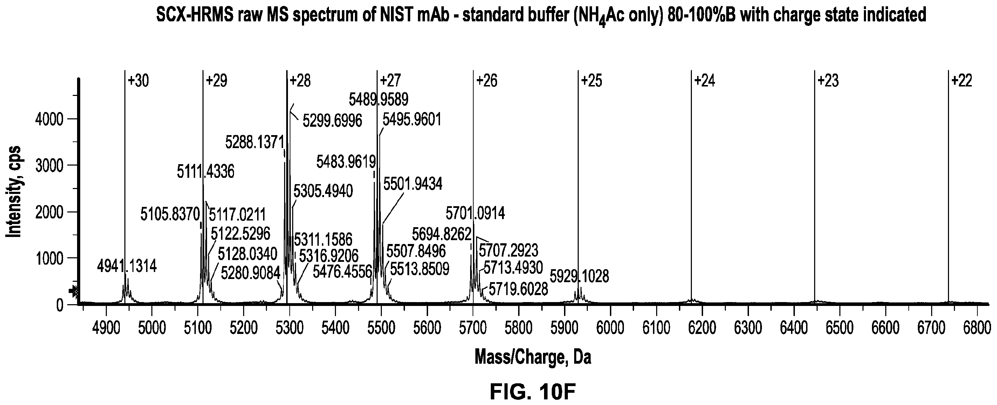

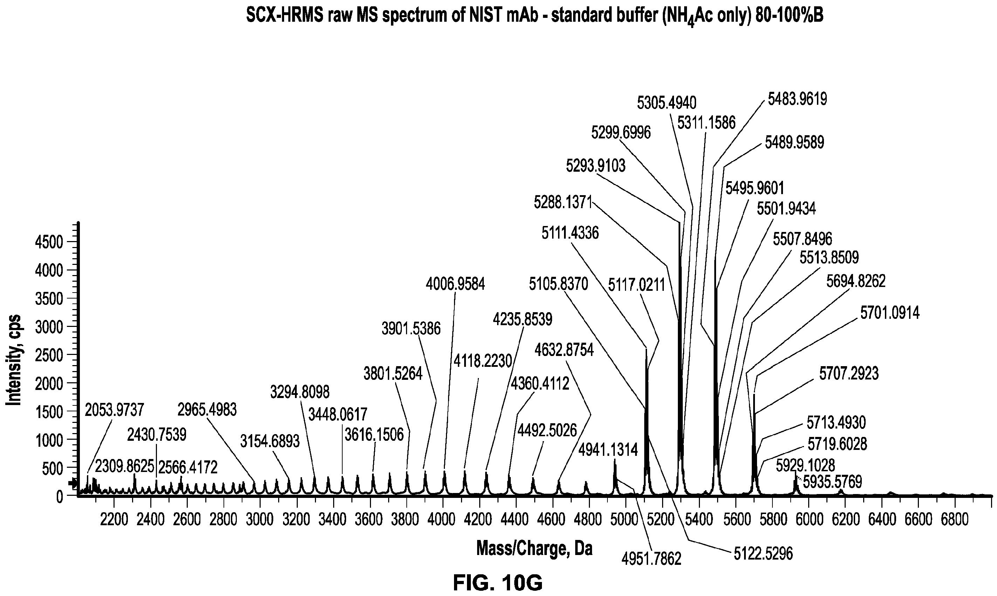

FIG. 10C shows a total ion current (TIC) chromatogram from the SCX-HRMS analysis of NIST mAb (10 mg/mL; 10 uL inj) using a strong cation exchange LC column (experimental 3 µm SCX, 100 x 2.1mm) with the standard buffer system containing NH4Ac only and a pH gradient of 80-100% B/10 min, 30 °C; +TOFMS (2000-7000), MS temp 500 °C. The dotted line forming a box within the major peak at about 4.5 min RT illustrates the approximate area for MS analysis shown in FIG. 10F and FIG. 10G.

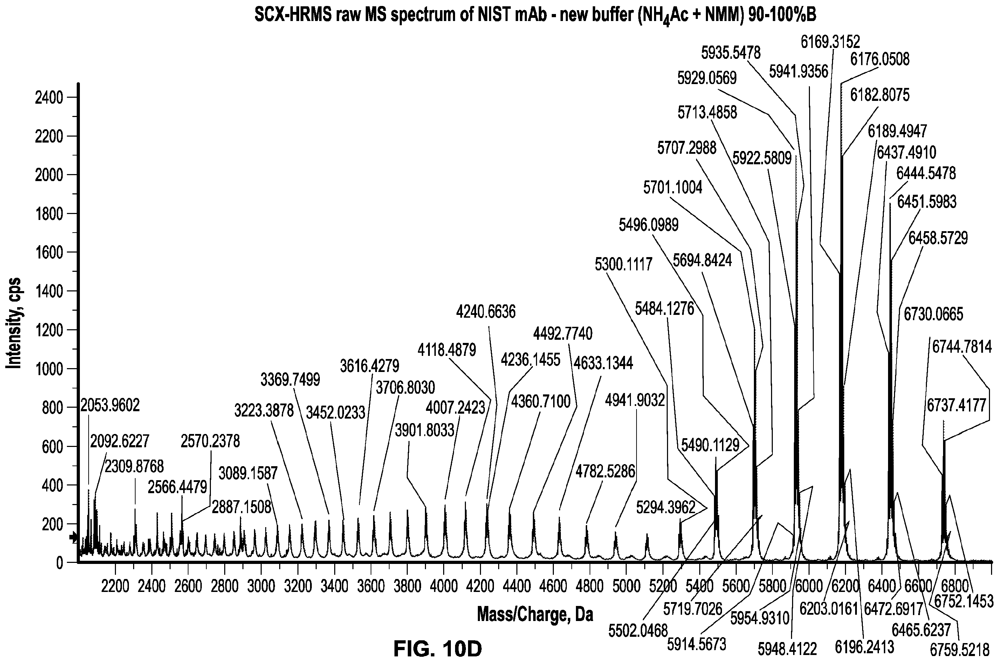

FIG. 10D shows a raw MS spectrum from the SCX-HRMS analysis of NIST mAb (10 mg/mL; 10 uL inj) using a strong cation exchange column (experimental 3 µm SCX, 100 x 2.1mm) with the new buffer system containing NH4Ac + NMM and a pH gradient of 90-100% B/10 min, +TOFMS(2000-7000) from 5.012 to 5.627 min. Mass/charge (m/z) data across the range from 2000 to 7000 Da is shown.

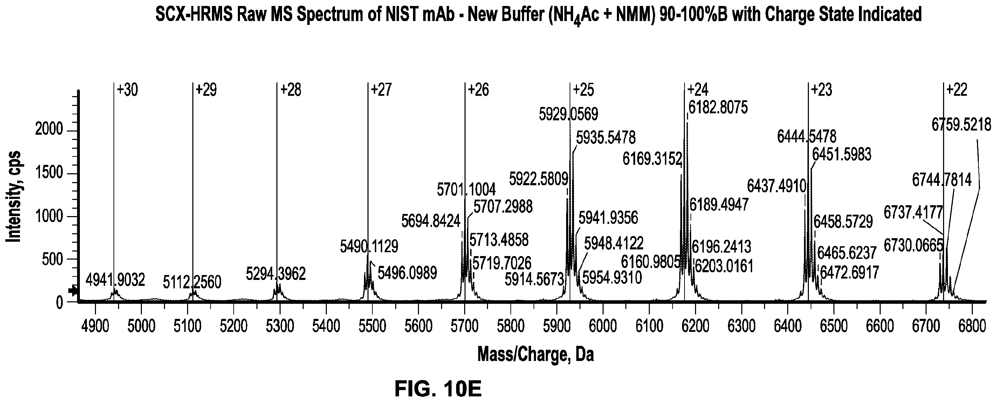

FIG. 10E shows a raw MS spectrum from the SCX-HRMS analysis of NIST mAb (10 mg/mL; 10 uL inj) using a strong cation exchange LC column (experimental 3 µm SCX, 100 x 2.1mm) with the new buffer system containing NH4Ac + NMM and a pH gradient of 90-100% B/10 min, +TOFMS (2000-7000), from 5.012 to 5.627 min. The m/z range displayed was narrowed to that giving the highest intensities for the native analyte (~4800-6900 Da) with this buffer system. Additionally, the charge of the analyte ions in the charge state envelope are indicated. With the new buffer system, the most intense MS peaks appear at higher masses/lower charges (+22 to +27) than seen with optimized standard NH4Ac buffer system (+25 to +30), as shown in FIG. 10F.
FIG. 10F shows a raw MS spectrum from the SCX-HRMS analysis of NIST mAb (10 mg/mL; 10 uL inj) using a strong cation exchange LC column (experimental 3 µm SCX, 100 x 2.1mm) with the standard buffer system containing NH4Ac with no NMM and a pH gradient of 80-100% B/10 min, +TOFMS (2000-7000), from 4.259 to 4.841 min.. The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at slightly lower masses/higher charges (+25 to +30), when compared to new NH4Ac + NMM buffer system (+22 to +27), as shown in FIG. 10E.
FIG. 10G shows a raw MS spectrum from the SCX-HRMS analysis of NIST mAb (10 mg/mL; 10 uL inj) from 4.259 to 4.841 min. using a strong cation exchange column (experimental 3 µm SCX, 100 x 2.1mm) with the optimized standard buffer system containing NH4Ac with no NMM and a pH gradient of 80-100% B/10 min, +TOFMS (2000-7000). Mass/charge range from 2000 to 6800 Da is shown.

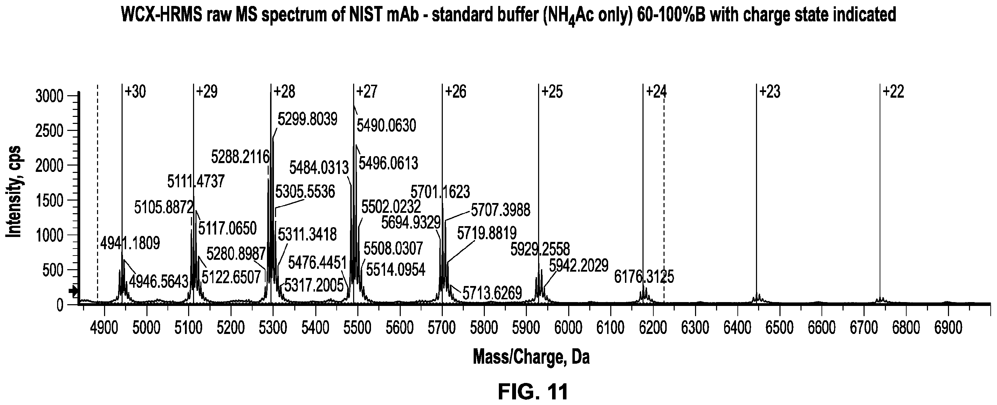

FIG. 11 shows a raw MS spectrum from the WCX-HRMS analysis of NIST mAb (10 mg/mL; 10 uL inj) using a weak cation exchange LC column (Phenomenex^{®} bioZen 6 µm WCX, 150 x 2.1mm) with the standard buffer system containing NH4Ac with no NMM and a pH gradient of 60-100% B, +TOFMS (2000-7000), 500 °C, from 4.772 to 5.012 min. The m/z range displayed was narrowed to that giving the highest intensities for the native analyte (~4800-7000 Da) with this buffer system. Additionally, the charge of the analyte ions in the charge state envelope are indicated. With the optimized standard buffer system, raw MS spectrum for NIST mAb using WCX-HRMS also exhibits the most intense MS peaks from +25 to +30, similar to SCX-HRMS raw spectrum shown in FIG. 10F.

### DETAILED DESCRIPTION

Monoclonal antibodies (mAbs) are an important and rapidly growing class of therapeutic proteins. Their inherent complexity and the wide variety of possible post translational modifications (PTMs) make effective characterization and quality monitoring challenging. Charge variants of mAbs commonly result from PTMs that occur during the manufacturing process. These PTMs, such as C-terminal lysine clipping and N-glycosylation, result in deletion/addition of basic and/or acidic residues relative to the native protein and thus impart differences to the overall charge of the molecule. Typically, analysis of mAbs and other biomolecule analytes is performed using a combination of liquid chromatography (LC) and mass spectrometry (MS). Several studies showing the utility of IEC for charge variant analysis (CVA) using high resolution mass spectrometry (HRMS) have been published. In the present disclosure, optimized MS compatible buffer systems were developed and used to improve control of pH gradients and thus resolution of charge variants of mAbs when using ion exchange chromatography (IEC) coupled online to HRMS. Different cation exchanger types (strong/weak), sorbent particle sizes, column lengths, flow rates, buffer systems, and gradient slopes were used. Methods and reagents have been developed using commercially available mAbs spanning a wide range of pI's. The disclosure provides improved buffer systems and methods for optimum performance in routine analyses.

Approaches to addressing problems with IEC-HRMS of protein analytes such as native monoclonal antibodies have been described, but each suffers from significant shortcomings.

Bailey et al., 2018 describe charge variant native mass spectrometry benefits mass precision and dynamic range of monoclonal antibody intact mass analysis. A MS-compatible method was sought to allow IEC separation with pH gradient elution. Mobile phase buffer A was 50 mM ammonium acetate (NH₄Ac), pH 6.6 without pH adjustment, buffer B was 50 mM ammonium acetate adjusted to pH 10.1 using ammonium hydroxide. Ammonium acetate provides buffering within +/- 1 pH unit of 4.75 (acetate pKa) and +/- 1 pH unit of 9.25 (ammonium pKa). Due to the broad gap between pKa values, pH gradients using ammonium acetate may suffer in terms of experimental linearity. Poor linearity of the pH gradient during the IEC separation was exhibited. Bailey et al., 2018 MABS, vol. 10, No. 8, 1214-1225. doi.org/ 10.1080/1940862.2018.1521131.

Fussl et al. 2018 performed charge variant analysis of monoclonal antibodies using direct coupled pH gradient cation exchange chromatography to high-resolution native mass spectrometry. Fussl et al., 2018, Anal Chem 90, 2018-4669. A volatile pH gradient buffer system with 25 mM ammonium bicarbonate, 30 mM acetic acid, pH 5.3(buffer A); and 10 mM ammonium hydroxide in 2 mM acetic acid, pH 10.8(buffer B) was employed using a MAbPac SCX-10 RS sulfonic acid strong cation exchange column with 5 micron particle size. After mixing, buffers were allowed to rest for 24 h at room temperature. The 24 h rest period was found to be problematic and the present inventors were not able to reproduce the author's results. Based on the composition and chemistry of the Fussl buffer, chemical changes (gas evolution, changes in concentration of "bicarbonate" and pH) may take place during the "resting" period. In addition, pH control was difficult, particularly between pH 7 to 8 due to poor buffering capacity over this pH range.

Fussl et al., 2019 describe characterization of the heterogeneity of adalimumab via charge variant analysis hyphenated on-line to native resolution Orbitrap mass spectrometry. Buffer A consisted of 25 mM ammonium bicarbonate and 30 mM acetic acid in water (pH 5.3) and buffer B consisted of 10 mM ammonium hydroxide in water (pH 10.9). Buffers were prepared at 5X stock and stored for 2 weeks at 4 °C before use. The use of relatively low ionic strength eluents, which are important for MS detection, was said to result in low buffering capacity, which may compromise analysis of mAbs with high pI values. Fussl et al., 2019 MABS vol. 11, No. 1, 116-128, doi.org/10.1080/19420862.2018.1531664.

pH gradient buffer systems are also commercially available. For example, CX-1 pH gradient buffers (Thermo Fischer Scientific) contain four zwitterionic buffer salts, namely (2-(N-morpholino)ethanesulfonicacid, 3-(N-morpholino)propanesulfonic acid, N-Tris(hydroxymethyl)methyl-3-aminopropanesulfonic acid and 3-(cyclohexylamino)-2-hydroxy- 1-propanesulfonic acid) in addition to NaCl and NaOH. It is assumed that this buffer system provides a pH gradient from pH = 5.6 to 10.2 and that it is concomitantly tuned to provide a slight ionic strength gradient. The BioResolve CX pH buffers (Waters) also include four buffer salts, including succinic acid, Bis-Tris propane, triethanolamine and N-cyclohexyl-3-aminopropanesulfonic acid and are assumed to provide a pH gradient from pH = 5.0 to 10.2. Farsang et al., 2020 J Chromatography A, vol. 1626, 30 Aug 2020, 461350.

The present disclosure provides methods and compositions for separating and/or characterizing an analyte in a sample. The methods may comprise liquid chromatography (LC). The LC may involve any appropriate LC method. The LC method may include analytical LC for characterization of the analyte. The LC method may include process LC for the separation and/or purification of the analyte. For example, the LC may include UHPLC, HPLC, ion-exchange chromatography (IEC), size-exclusion chromatography (SEC), hydrophilic interaction chromatography (HILIC), and/or reversed-phase chromatography (RPLC). Therefore, a liquid chromatography column, ultra-performance chromatography column, high-performance chromatography, ion-exchange chromatography column, size-exclusion chromatography column, hydrophilic interaction chromatography column, reverse phase chromatography column may be used in methods of the disclosure.

The methods described herein may further comprise additional processes upstream or downstream such as affinity chromatography (e.g., protein A or Protein G), anion exchange chromatography (AEC), hydrophobic interaction chromatography (HIC), low pH viral inactivation, viral filtration, ultrafiltration, and/or diafiltration

The liquid chromatography (LC) may be coupled to a mass spectrometer. The liquid chromatography may be coupled directly to a mass spectrometer.

The analyte may be a biomolecule such as a monoclonal antibody (mAb) and/or a charge variant thereof. One of the most common LC techniques used to characterize charge variants in mAbs is ion-exchange chromatography (IEC).

### Definitions

The terminology used herein is for the purpose of describing particular embodiments only and is not intended to be limiting of the disclosure.

The singular forms "a", "an" and "the" are intended to include the plural forms as well, unless the context clearly indicates otherwise.

The term "and/or" refers to and encompasses any and all possible combinations of one or more of the associated listed items.

The term "about," when referring to a measurable value such as an amount of a compound, dose, time, temperature, and the like, is meant to encompass variations of ±10%, 5%, 1%, 0.5%, or even 0.1% of the specified amount.

The terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms, including technical and scientific terms used in the description, have the same meaning as commonly understood by one of ordinary skill in the art to which this disclosure belongs. In the event of conflicting terminology, the present specification is controlling.

The term "room temperature" refers to a temperature of 23 °C (293-298 K, 71 °F) and an absolute pressure of 1 atm (14.696 psi, 101.325 kPa), unless otherwise specified.

The term "isomer" refers to two compounds having same chemical formula but a different arrangement of atoms in the molecule.

The term "analog" or "structural analog" refers to a compound having a molecular structure similar to that of another compound, but differing in respect to a certain component.

The embodiments described in one aspect of the present disclosure are not limited to the aspect described. The embodiments may also be applied to a different aspect of the disclosure as long as the embodiments do not prevent these aspects of the disclosure from operating for its intended purpose.

Cation exchange chromatography is a form of ion exchange chromatography (IEX) that may be used to separate molecules based on their net surface change. Cation exchange chromatography employs a negatively charged ion exchange resin with an affinity for molecules with a net positive surface charge.

A protein's net surface charge may change with pH as determined by the protein's isoelectric point (pI). At a pH equal to the pI, the protein will carry no net charge. At a pH below the pI, the protein will carry a net positive charge. At a pH above the pI, the protein will carry a net negative charge. A protein's pI may be calculated by its primary amino acid sequence. A negatively charged cation exchange resin may be selected when the protein of interest carries a net positive charge at the working pH. The buffer pH will influence the protein's net surface charge. Because the protein may not be stable at every pH, protein stability and buffer choice may help determine the appropriate IEX media for purification.

Cation exchange chromatography column resins may be classified by the pKa of the functional group on the resin. Those resins containing strong acids or bases are termed "strong" ion exchangers while those containing weak acids or bases are termed "weak" ion exchangers.

The term "strong cation exchange" (SCX) or "strong cation exchange resin" or "strong cation exchange stationary phase" refers to an ion exchange resin comprising a sulfonic acid functional group, such as a methyl sulfonate functional group (e.g., -OCH₂CH₂OH-CH₂-O-CH₂-CHOH-CH₂-SO₃⁻) which may be designated S, or sulphonyl functional group (e.g., -CH₂-CH₂-CH₂SO₃⁻), which may be designated SP. A typical pH range for S strong cation exchange resin may be from pH 2-12. A typical pH range for SP strong cation exchange resin may be from pH 2-14.

The term "weak cation exchange" (WCX) or "weak cation exchange resin" or "weak cation exchange stationary phase" refers to an ion exchange resin typically comprising a carboxylic acid functional group, such as a carboxymethyl functional group (e.g., -O-CH₂-CO₂⁻) which may be designated CM, or other carboxylic acid functional group (-CO₂⁻) which may be designated C.

The term "SEC" refers to size-exclusion chromatography. Size-exclusion chromatography (SEC) involves separating molecules based on size from largest to smallest in proportion to their molecular size in solution. Very large molecules are excluded from the packed bed and are eluted first, in the void volume. Smaller molecules may penetrate pores to various degrees depending on size. With the smallest molecules diffusing furthest into pores and eluting last. Different mobile phases may effect elution order because of changes in size in solution, for example, due to hydrodynamic radius or radius of gyration. SEC may be used to separate proteins such as monoclonal antibodies from aggregates, fragments, conjugates, excipients, impurities, and the like. SEC may be used for size-based separations of biological compounds. For example, SEC is appropriate for aggregate and fragment analysis in the research, development, and manufacturing of biotherapeutic molecules.

The term "m/z" in a mass spectrum represents the mass-to-charge ratio of an individual molecule, representing the exact isotopic composition of each molecule. The most intense peak arises from the most likely composition of isotopes.

The term "charge envelope" or "charge state envelope" refers to a group of peaks in LC/MS raw data, for example, of an intact mAb. Proteins form multiply charged ions during electrospray ionization. Large proteins such as monoclonal antibodies have a charge distribution envelope, or "charge envelope" or "charge state envelope." For example, using the new buffer system for trastuzumab, the center of the envelope is charge +24 as shown in FIG. 6E. This is compared to standard buffer system for trastuzumab, where the center of the envelope is charge +27 as shown in FIG. 6F. Multiple peaks are observed, and these are due to different glycoforms or charge variants of the mAb. The charge states of native proteins in ESI-MS are a complex function of several parameters and the relative importance of these are still debated. Of the different parameters debated, the conformation of the protein in solution (solvent accessible surface area), the protein sequence (number of charged AA at "surface" of protein, solvent accessible AAs) and the gas phase basicity of the components in the ionization environment are typically considered important. The charge state of an analyte is important with MS detection of biomacromolecules since lower charge states can give higher resolution in the deconvoluted mass spectrum. Lower charge states are generally preferred in the native MS of biomolecules since this indicates reduced protein denaturing ("more native" conformation) or fewer adducts or both. In some embodiments, the new buffer system results in a lower charge state envelope in the raw mass spectrum of a protein analyte by at least about 3 or at least about 2 charge states, compared to the standard buffer system.

Detection of the LC eluate may be by any appropriate method. The eluate may be monitored by UV detection, for example, by measurement of absorbance at a preselected wavelength with a UV detector, and/or in a UV range, for example, using a diode-array detector (DAD). UV detection may comprise monitoring the eluate by absorbance at any appropriate UV wavelength. For example, UV detection of protein amide bond (220 nm), off-peak (230 nm), protein aromatic amino acid side chains of Tyr, Phe, Trp (280 nm), or nucleic acids (260 nm), or any other appropriate UV wavelength value in between. The LC eluate may also be monitored by mass spectrometry (MS), refractive index (RI), laser-light scattering (LS), fluorescence detector (FL), or any other appropriate detection method. A pH meter may also be employed to monitor effluent pH response provided by the buffer system online

The term "resolution" refers to the measure of how well two peaks are separated. Resolution can be determined by Rₛ=(tᵣ,2-tᵣ,1)/(0.5x (w₁+w₂)), wherein tᵣ is the retention time of either peak 1 or peak 2, and w₁ is the peak width at half height for peak 1 or peak 2. In a similar fashion, resolving power and peak capacity are used to refer to the measure of how many peaks can fit within a given separation space.

Peak capacity may be determined as Pc=1+(t/w_{avg}), wherein t is the time corresponding to the given separation space and w_{avg} is the average peak width at half height observed for peaks in a given separation.

Total ion current ("TIC") chromatogram peak height and TIC peak signal-to-noise ratio are two values that may be used to define the sensitivity of an LC-MS analysis. TIC signal-to-noise may be impacted by selection of mobile phase.

In addition to chromatographic resolution, LC-MS methods may be evaluated by the quality of the mass spectra they provide.

The term "eluent" is the buffer or buffer mixture used in chromatography and is synonymous with mobile phase.

The term "eluate" refers to the solution containing the analyte and solvent emerging from the chromatography column. The eluate occurs during the course of a separation.

The term "effluent" refers to the stream emerging from the chromatography column, whether there is a separation taking place or not.

Mass spectrometry ("MS") is an analytical technique that measures the mass-to-charge ratio of a charged molecule or molecule fragments formed from a sample. MS may be used to analyze the mass, chemical composition, and/or chemical structure of a sample of interest. In general, MS includes three steps: ionizing a sample to form charged molecules or molecule fragments (i.e., ions); separating the ions according to their mass-to-charge ratio; and detecting the separated ions to form a mass-to-charge signal (i.e., spectrum). The formation of the ions can be achieved through routine MS ionization techniques, such as, for example, Electrospray ionization (ESI), Fast Atom Bombardment (FAB), Chemical Ionization (CI), Electron Impact (EI), Atmospheric Solids Analysis Ionization (ASAI), Atmospheric Pressure Photoionization (APPI), Desorption Electrospray Ionization (DESI), Atmospheric Pressure Vapor Source (APVS), Matrix-Assisted Laser Desorption/Ionization (MALDI). There are many different types of MS devices. For example, sector, time-of-flight (TOF), quadrupole, ion trap, Fourier transform ion cyclotron resonance, and tandem (two or more of the above combined in tandem or orthogonal) mass spectrometers are all different instruments that are considered to be MS devices. Certain characteristics of MS analysis include, e.g., mass accuracy, resolution, sensitivity, dynamic range, selectivity, and specificity, etc.

The mass spectrometry (MS) may comprise sample preparation and introduction, ion formation, mass separation, and data processing.

The MS ion formation may comprise, e.g., electrospray ionization (ESI) or matrix assisted laser desorption ionization (MALDI). In electrospray ionization (ESI), a solution containing the sample of interest is nebulized into fine droplets. The droplets, dry, reduce in size, and divide into smaller droplets, eventually generating desolvated analyte ions with one or more residual charges. Proteins tend to form multiply charged ions during electrospray analysis, facilitating analysis by mass spectrometry. The mass separation may include, e.g., quadrupole, TOF, Orbitrap.

The MS data processing may include deconvolution of the raw MS spectrum. For example, ESI MS mass spectrum may be subjected to mathematical deconvolution to convert raw mass spectrum comprising m/z to protein analyte mass data. For example, $m / z = mass to charge ratio$
Mᵣ = mass of protein
n = # of charges on m/z
mₚ = mass of H+ = 1.0073
Given m/z₁ = (Mr + n₁mₚ)/n₁
And n₂= n₁ +1
Can rearrange to solve for n₁: ${n}_{1} = \left(m/{z}_{2}-{m}_{p}\right) / \left(m/{z}_{1}-m/{z}_{2}\right)$ and ${calcMr}_{1} = \left({n}_{1}∗m/{z}_{1}\right) - \left({n}_{1}∗1.0073\right) = {Mr}_{1}$ ${calcMr}_{2} = \left({n}_{2}*m/{z}_{2}\right) - \left({n}_{2}*1.0073\right) = {Mr}_{2}$

For example, see Covey et al., The determination of protein, oligonucleotide and peptide molecular weights by ion-spray mass spectrometry, Rapid Commun. Mass Spectrom. 2:11, 1988. Computer assisted deconvolution of raw mass spectra may also be performed. Mann, M.; Meng, C.K.; Fenn, J.B. Interpreting Mass Spectra of Multiply Charged Ions. Anal. Chem. 1989, 61, 1702-1708; Labowsky, M.; Whitehouse, C.M.; Fenn, J.B. Three-Dimensional Deconvolution of Multiply Charged Spectra. Rapid Commun. Mass Spectrom.1993, 7, 71-84; Zhang, Z.; Marshall, A.G. A Universal Algorithm for Fast and Automated Charge State Deconvolution of Electrospray Mass-to-Charge Ratio Spectra. J. Am. Soc. Mass Spectrom.1998, 9, 225-233.

MALDI is a soft ionization technique appropriate for native protein analysis, such as native monoclonal antibody MS.

ESI and MALDI ion formation processes are competitive-different compounds can have large variations in ionization efficiency. Ion suppression can be caused by salt, detergents, polymers. The protein analyte may be separated from interfering materials prior to the ionization step. Allowable concentrations of various salts and buffer components have been described by the Harvard Center for Mass Spectrometry. https://massspec.fas.harvard.edu/files/smms/files/saltbuffer. Nonvolatile salts (e.g., NaCl) can cause suppression of intact proteins leading to poor detection sensitivity, so it is important to minimize the introduction of these compounds into the MS.

Traditionally in order to avoid MS ion suppression, offline sample cleanup and desalting techniques have been utilized such as dilution, dialysis/buffer exchange, zip-tip, molecular weight cutoff (MWCO) filters, and/or desalting columns. For example, BioRad Micro Biospin P-6 gel columns, remove small molecules, salt, with a MWCO 6000 Da. Drawbacks of offline sample cleanup include requirement for > 10uL of sample and/or high concentrations, time and sample for method development to minimize protein loss due to precipitation, failure to stick to or to elute from media, and decomposition of analyte protein. Offline desalting/cleanup may not be feasible if the sample is limited with no prior optimization. Typical online desalting techniques for LC-MS may include, for example, reverse phase (RP) chromatography, size exclusion or capillary electrophoresis. RP desalting columns may utilize for example, phenyl, diphenyl, polyphenyl, C4, C8, or polymeric reverse phase (PLRP-S). The RP on-line prep columns will retain proteins allowing the salts to be washed to waste prior to protein elution into the mass spectrometer. However, use of desalting columns may increase expense and cycle times by at least several minutes for intact antibodies. The present disclosure provides a single mobile phase buffer system that is suitable for IEC- HRMS, comprising low salt concentration to avoid MS ion suppression, providing reproducible pH gradient elution, and is amenable to both UV and MS detection. Methods comprising direct coupled pH gradient cation exchange chromatography to high resolution mass spectrometry are provided.

In some embodiments, the mobile phase buffer compositions of the disclosure may contain no more than 50 mM, no more than 30 mM, no more than 25 mM salt concentration, no more than about 20 mM salt concentration, or no more than about 15 mM salt concentration. In some embodiments, compositions of the disclosure may contain 2-25 mM, 2-20 mM, or 2-15 mM salt concentration. The salt may be an ammonium carboxylate salt. The ammonium carboxylate may be an ammonium C₁-C₂ carboxylate. The ammonium carboxylate may be ammonium acetate or ammonium formate. In some embodiments, the compositions of the disclosure may contain 2 mM to 25 mM, 2 mM to 20 mM, or 2 mM to 15 mM ammonium acetate concentration. In some embodiments, the compositions of the disclosure may contain no more than 25 mM, no more than 20 mM, or no more than about 15 mM ammonium acetate concentration.

In some embodiments, the mobile phase buffer composition further comprises from about 1 mM to about 50 mM, about 1 mM to about 25 mM, about 1 mM to about 20 mM, about 1 mM to about 10 mM, from about 1 mM to about 6 mM, or from about 2 mM to about 5 mM N-methylmorpholine (NMM), or an isomer thereof, or an analog thereof.

Isomers of N-methylmorpholine may include 2-methylmorpholine, 2-methylmorpholine, 2-methyl-1,3-oxazinane, 3-methyl-1,3-oxazinane, 4-methyl-1,3-oxazinane, 5-methyl-1,3-oxazinane, and 6-methyl-1,3-oxazinane.

Analogs of N-methylmorpholine may include any appropriate alkyl or dialkyl morpholine or alkyl or dialkyl 1,3-oxazinane. The analog of N-methylmorpholine may be selected from the group consisting of an alkyl C₁₋C₆ morpholine, a dialkyl C₁-C₆ morpholine, an alkyl C₁₋C₆- 1,3-oxazinane, and a dialkylC₁₋C₆- 1,3-oxazinane.

Analogs of N-methyl morpholine may include an alkylmorpholine compound. The alkylmorpholine compound may include alkyl C₁-C₆ morpholine compounds, alkyl C₁₋C₄ morpholine compounds, or alkyl C₁₋C₃ morpholine compounds. The alkyl morpholine may be, for example, ethylmorpholine, propylmorpholine, isopropylmorpholine, butylmorpholine, or isobutylmorpholine, and the like.

Analogs of N-methyl morpholine may include an N-alkylmorpholine compound. The N-alkylmorpholine compound may include N- alkyl C₁-C₆ morpholine compounds, N- alkyl C₁₋C₄ morpholine compounds, or N- alkyl C₁₋C₃ morpholine compounds. The N-alkyl morpholine may be, for example, N-ethylmorpholine, N-propylmorpholine, N-isopropylmorpholine, N-butylmorpholine, or N-isobutylmorpholine, and the like.

Analogs of N-methyl morpholine may include a dialkylmorpholine. The dialkylmorpholine compound may include dialkyl C₁₋C₆ morpholine compounds, dialkyl C₁₋C₄ morpholine compounds, or dialkyl C₁₋C₃ morpholine compounds. The dialkylmorpholine may be a 2,2-dialkylmorpholine, 2,3-dialkylmorpholine, 2,4-dialkylmorpholine, 2,5-dialkylmorpholine, 2,6-dialkylmorpholine, 3,3-dialkylmorpholine, 3,4-dialkylmorpholine, or 3,5- dialkylmorpholine, or the like.

For example, the dialkyl morpholine may be a 2,2-dimethylmorpholine, 2,3-dimethylmorpholine, 2,4- dimethylmorpholine, 2,5-dimethylmorpholine, 2,6-dimethylmorpholine, 3,3-dimethylmorpholine, 3,4-dimethylmorpholine, or 3,5-dimethylmorpholine, or the like

The dialkyl morpholine or dialkyl-1,3-oxazinane may be in any configuration. Certain dialkyl morpholine or 1,3-oxazinane compounds may be in cis configuration, trans configuration, or a mixture of cis- and trans-configurations. For example, cis,trans-2,6-dimethylmorpholine, cis -2,6-dimethylmorpholine, or trans-2,6-dimethylmorpholine, or any combination thereof.

Analogs ofN-methyl morpholine may include an alkyl 1,3-oxazinane compound.

The alkyl 1,3-oxazinane compound may include alkyl C₁₋₆ -1,3-oxazinane compounds, alkyl C₁₋₄ -1,3-oxazinane compounds, alkyl C₁₋₃ -1,3-oxazinane compounds, N-alkyl C₁₋₆ -1,3-oxazinane compounds, N- alkyl C₁₋₄ -1,3-oxazinane compounds, or N- alkyl C₁₋₃ -1,3-oxazinane compounds.

For example, the N-alkyl 1,3-oxazinane may include N-methyl-1,3-oxazinane, N-ethyl-1,3-oxazinane, N-propyl-1,3-oxazinane, N-isopropyl-1,3-oxazinane, N-butyl-1,3-oxazinane, N-isobutyl-1,3-oxazinane, and the like.

Analogs ofN-methyl morpholine may include a dialkyl 1,3-oxazinane compound. Isomers of N-methyl morpholine may include a dialkylC₁₋C₆- 1,3-oxazinane, dialkylC₁₋C₄- 1,3-oxazinane, or dialkylC₁₋C₃- 1,3-oxazinane. The dialkyl 1,3-oxazinane may be a 2,4- dialkyl-1,3-oxazinane, 2,5- dialkyl-1,3-oxazinane, 2,6-dialkyl-1,3-oxazinane, 3,5- dialkyl-1,3-oxazinane, 3,4- dialkyl-1,3-oxazinane, 2,2-dialkyl-1,3-oxazinane, or 3,3- dialkyl-1,3-oxazinane. For example, the dialkyl 1,3-oxazinane may be a 2,4- dimethyl-1,3-oxazinane, 2,5- dimethyl-1,3-oxazinane, 2,6-dimethyl-1,3-oxazinane, 3,5- dimethyl-1,3-oxazinane, 3,4- dimethyl-1,3-oxazinane, 2,2- dimethyll-1,3-oxazinane, or 3,3- dimethyl-1,3-oxazinane.

The term "alkyl" may refer to C₁₋₆ alkyl, or C₁₋₄ alkyl, or C₁₋₃ alkyl, or C₁₋₂ alkyl. The alkyl may be straight chain or branched alkyl. For example, the alkyl may be methyl, ethyl, propyl, isopropyl, butyl, sec-butyl, isobutyl, pentyl, hexyl, and the like.

The "analyte" may be a biomolecule. The analyte can be a protein, a peptide, a glycan or a combination thereof. The analyte can include multiple proteins, multiple peptides, multiple glycans, or combinations thereof. The protein may be an antibody, an antigen-binding fragment thereof, or an antibody-drug conjugate (ADC). The biomolecule analytes may be recombinant, synthetic, or isolated biomolecules. The biomolecule may be a protein analyte.

The term "protein" as used herein, refers to a polymeric chain of amino acids called polypeptides. A protein may also include a number of modifications, including post-translational modification (PTM), phosphorylation, lipidation, prenylation, sulfation, hydroxylation, acetylation, addition of carbohydrate (glycosylation and glycation), addition of prosthetic groups or cofactors, formation of disulfide bonds, proteolysis, assembly into macromolecular complexes and the like. The antibody may be a monoclonal antibody (mAb) or a polyclonal antibody, or an antigen-binding fragment thereof. The antibodies may be full-length antibody (for example, an IgG1 or IgG4 antibody), bispecific antibody (bsAb), or may comprise only an antigen-binding portion (for example, a Fab, F(ab')2, Fab₃, scFv, bis-scFv, minibody, diabody, tetrabody, triabody, fragment), and may be modified to affect functionality, may be monovalent, divalent, trivalent, tetravalent, and have a higher valency, or may be an antibody-conjugate. Antigen-binding fragments may include, e.g.: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. The biomolecule analytes may be native mAbs and charge variants thereof.

### Compositions

Aqueous mobile phase buffer systems are provided for use in liquid chromatography optionally directly coupled to high-resolution mass spectrometry in the analysis of intact biomolecules, such as monoclonal antibodies. In some embodiments, the LC is ion-exchange chromatography (IEC) or size-exclusion chromatography (SEC).

The new mobile phase buffer systems provided herein are suitable for both UV and MS detection because all components are volatile, and none have significant UV absorbance at UV wavelengths commonly used for protein analysis. The new mobile phase buffer system comprises a low salt concentration of no more than about 25 mM and is suitable for use in pH gradient elution in IEC directly coupled to HRMS.

The new mobile phase buffer system provides optimal pH control, sensitivity, and resolution in analysis of native biomolecules, such as monoclonal antibodies (mAbs). Buffer compositions and pH values have been optimized for mAb charge variant analysis. Gradient range and slope may be optimized for each analyte, for example, a native mAb. MS conditions may be optimized to maximize sensitivity and/or minimize adducts. Ingredients used for the buffer components are preferably high purity, trace metal grade to improve sensitivity and reduce spectral complexity caused by metal adducts.

Buffer pH and ionic strength are important for all forms of ion exchange chromatography. Typically, the buffer pH may be adjusted after the salt concentration has been adjusted. Because buffer pH and ionic strength greatly affect protein binding to the column resin, it is important to be sure the pH is appropriately adjusted and appropriate counterions are used.

The technologies described herein solve problems discovered in prior art such as non-linearity of pH gradient elution and poor chromatographic reproducibility, when using ion exchange chromatography coupled directly to high-resolution mass spectrometry (online HRMS).

The technologies described herein solve problems discovered in the prior art including poor MS sensitivity due to excessive salt concentration in mobile phase buffer in ion exchange chromatography with HRMS; it has been discovered that typically ~40 mM or greater salt concentration may reduce MS sensitivity. The present disclosure provides a buffer system having highest buffer capacity in combination with the lowest ionic strength appropriate for use in HRMS. In some embodiments, an ionic strength of 25 mM or less salt concentration is employed.

The technologies described herein solve problems discovered in prior art such as poor chromatographic resolution of charge variants of intact monoclonal antibodies (mAbs), especially low isoelectric point (pI) mAbs (e.g., Infliximab, pI 7.6), when using ion exchange chromatography with high-resolution mass spectrometry (HRMS).

The technologies described herein solve problems discovered in the prior art including reduced mass spectrometric (MS) resolution due to high charge states of intact proteins in IEC-HRMS, i.e., MS spectra indicative of significant protein denaturing.

The new mobile phase buffer system buffer system provided herein may be used for pH gradient based chromatographic separations with mass spectrometric (MS) detection (i.e., MS compatible) giving 1) improved pH control, 2) improved MS sensitivity, and 3) increased mass spectral resolution (higher mass spectrum charge envelope for intact proteins indicating "more-native" state), such as charge variant analysis of intact monoclonal antibodies using ion exchange chromatography.

Other advantages of the improved buffer system provided herein include better chromatographic and mass spectrometric resolution of intact mAb charge variants. These are often critical quality attributes that must be identified and characterized during the development and manufacture of biotherapeutics.

The improved buffer systems may be utilized with either weak ion exchange stationary phase or strong ion exchange stationary phase materials and columns.

In particular, the new pH gradient buffer system components have pKa values that span the pH range of primary interest for biomolecules such as mAbs, i.e., in a range of from pH 4.5-10.8, from pH 4.5-10.5, from pH 5-10.5, or from pH 5.2-10.2. For example, Acetate pKa = 4.8, N-methylmorpholine pKa = 7.4, ammonia pKa = 9.2. One reason N-methylmorpholine was selected for use in the new buffer system is the intermediate pKa of ~7.4, which makes the pH gradient more linear and more reproducible.

The new buffer system is also UV compatible, which is a further benefit of using N-methylmorpholine vs other weak bases. The N-methylmorpholine may be in the base form. Other potential MS compatible weak bases (e.g., pyridine based) with pKa close to the range desired have significant UV absorption. N-methylmorpholine has good transparency at the UV wavelengths commonly used for biotherapeutics (280nm) allowing the same buffer to be used for both MS and UV detection. This is beneficial because the retention time/elution order of all peaks (including impurities) are typically identified using MS, then routine monitoring with UV detection is performed using the retention times (RTs)/elution order determined with MS. Changing the buffer used for each detector could greatly complicate this task.

Biomolecule (e.g., protein, peptide, and/or glycan) LC-MS is not merely about achieving high sensitivity detection. The capability of the method is greatly impacted by the resolution of the chromatography.

The disclosure provides pH gradient buffer systems appropriate for use in LC separation and optionally MS of biomolecule analytes.

For example, development of the present pH gradient buffer system involved analysis of 5 intact native monoclonal antibodies spanning the range of relevant pIs including Infliximab pI 7.6 (e.g., Remicade), Cetuximab pI 8.8 (e.g., Erbitux), trastuzumab pI 9.1 (e.g. Kanjinti), NIST reference mAb pI 9.2, and Rituximab pI 9.4 (e.g., Rituxan). The buffer systems provided herein are appropriate for use in weak ion exchange chromatography and/or strong ion exchange chromatography. For example, the buffer systems provided herein are appropriate for separation of analytes such as charge variants of native mAbs using a weak ion exchange column, such as Phenomenex^{®} bioZen 6um WCX, or a strong ion exchange column, such as ThermoScientific^{™} mAbPac^{™} 5um SCX.

The inventive buffer system may comprise a ready-to-use buffer concentration of <25 mM ammonium acetate, from about 2 to about 25 mM ammonium acetate, from about 2 to 20 mM ammonium acetate, or from about 5 mM to about 15 mM ammonium acetate. The inventive mobile phase buffer system may comprise a ready-to-use buffer concentration of from about 1 mM to about 10 mM N-methylmorpholine, from about 2 mM to about 8 mM N-methylmorpholine, or from about 2 mM to about 5 mM N-methylmorpholine. The inventive buffer system may comprise a ready-to-use buffer concentration of <25 mM ammonium acetate, ≤20 mM ammonium acetate, from about 2 to 25 mM ammonium acetate, from about 2 to about 20 mM ammonium acetate, from about 2 to about 15 mM ammonium acetate, or from about 5mM to about 15 mM ammonium acetate and from about 1 mM to about 10 mM N-methylmorpholine, from about 2 mM to about 8 mM N-methylmorpholine, or from about 2 mM to about 5 mM N-methylmorpholine. The buffer system may comprise a pH in the range of, or spanning the range of, from about pH 4.5 to about pH 10.5, from pH 5-10.5, or from pH 5.2-10.2.

The disclosure provides an aqueous buffer system comprising one or more, two or more, or three or more parts. The buffer system may comprise a mobile phase comprising a two part aqueous buffer system. The two part aqueous buffer system may comprise a part A buffer and a part B buffer. In some embodiments, the part A of the aqueous buffer system may comprise from about 10 mM to about 25 mM ammonium acetate, and from about 2 mM to about 10 mM N-methylmorpholine, at a pH of from about pH 4.5 to about pH 5.5 adjusted with acetic acid. In certain embodiments, part A of the aqueous buffer system may comprise from about 10mM to about 20 mM ammonium acetate, and from about 3 mM to about 8 mM N-methylmorpholine, at a pH of from about pH 5 to about pH 5.5 adjusted with acetic acid. In some embodiments, the part B of the aqueous buffer system may comprise from about 2 mM to about 10 mM ammonium acetate, and from about 1 mM to about 5 mM N-methyl morpholine, at a pH 9 to about 10.5, adjusted with ammonium hydroxide. In certain embodiments, the part B of the aqueous buffer system may comprise from about 3 mM to about 8 mM ammonium acetate, and from about 1 mM to about 3 mM N-methyl morpholine, at a pH 9.5 to about 10.5, adjusted with ammonium hydroxide. The two-part aqueous buffer system may be designed to span the pH range of from pH 4.5-10.5, from pH 5-10.5, or from pH 5.2-10.2. A broader pH range of from about pH 3.8-10.8 may be employed.

The mobile phase additives may include no more than about 100 ppb of any individual metal impurity. In other words, each metal impurity contained in the mobile phase additive is not present in an amount greater than about 100 ppb. In some embodiments, the mobile phase additives each comprise less than about 90 ppb, 80 ppb, 70, ppb, 60, ppb, 50 ppb, 40 ppb, 30 ppb, 20 ppb, or 10 ppb of any individual metal impurity. In some embodiments, the mobile phase additives each comprise less than about 95 ppb, 85 ppb, 75, ppb, 65, ppb, 55 ppb, 45 ppb, 35 ppb, 25 ppb, or 15 ppb of any individual metal impurity. The metal impurity is any metal that affects the desirable features of a mass spectrum, e.g., the quality of the mass spectrum. The metal impurity can be, for example, sodium, potassium, calcium, nickel, copper, and/or iron. These aspects of the technology extend to the ready-to-use mobile phase or any concentrates thereof subsequently prepared with the above described mobile phase additive.

The buffer system may employ deionized water (wt/wt or vol/vol) using LC/MS grade water, MS grade water, or Type 1 reagent grade water with a specific resistance of about 18.2 megohm-cm filtered through a 0.2 micron filter.

The water may have trace impurities ≤20ppb. Like the mobile phase additives, the water may have less than about 100 ppb of any individual metal impurity, or less than about 95 ppb, 85 ppb, 75, ppb, 65, ppb, 55 ppb, 45 ppb, 35 ppb, 25 ppb, or 15 ppb of any individual metal impurity.

The mobile phase may have less than about 100 ppb, 90 ppb, 80 ppb, 70 ppb, 60 ppb, 50 ppb, 40 ppb, 30 ppb, 20 ppb, or 10 ppb of any individual metal impurity. The mobile phase may have less than about 50 ppb of any individual metal impurity. The mobile phase may have less than about 20 ppb of any individual metal impurity. The individual metal impurity, can be, for example, aluminum, barium, cadmium, calcium, chromium, cobalt, copper, iron, lead, magnesium, manganese, nickel, potassium, silver, sodium, tin, or zinc.

The compositions of the disclosure may be made commercially available in the form of ready-to-use buffers, concentrated buffers for dilution, as well as a component of a kit, such as a combined product comprised of a buffer according to the disclosure along with an LC column or device. The ready-to-use buffers or concentrated buffers for dilution may be stored in any appropriate container. The container should be clean and free of extractable material/residues. For example, the buffer storage container may be an appropriate plastic or a glass storage container. In some embodiments, use of a plastic container for buffer storage is preferred, for example, in order to reduce leaching of contaminants from glass. The container may comprise any appropriate plastic material. In some embodiments, the container may comprise high-density polyethylene (HDPE), polypropylene (PP), polytetrafluoroethylene (PTFE), or polystyrene (PS) plastic material. The container may be sealed, for example, to exclude contaminants.

The kit may include a chromatography column, a container having a volume of concentrated mobile phase, and instructions for use and/or a website address for instructions for use. The chromatography column has a stationary phase material inside the column. The stationary phase material can be any stationary phase material described herein, for example, an ion-exchange stationary phase, a size-exclusion stationary phase, a hydrophilic interaction stationary phase, and/or reversed-phase stationary phase.

The mobile phase composition may be a liquid in the form of a ready-to-use buffer or in a concentrated buffer. The concentrated buffer may be a 2X, 3X, 4X, 5X, 6X, 7X, 8X, 9X, 10 X, 11X, 12X, 13X, 14X, 15X, 16X, 17X, 18X, 19X, or 20X or more concentration for dilution prior to use, or any value in between. In some embodiments, the mobile phase buffer is a 10X concentrate. Optionally, an organic solvent such as acetonitrile or isopropanol may be employed at a concentration of up to 10% v/v. The concentrate may be diluted to prepare a 1X buffer system. In some embodiments, the mobile phase buffer is a ready-to-use mobile phase buffer composition.

The instructions for use may instruct the user to obtain a sample containing at least one biomolecule (e.g., a protein such as a mAb process sample or mAb purified sample) in a sample matrix, as well as to dilute the mobile phase concentrate with an appropriate water to obtain from about 2 mM to about 25 mM ammonium acetate, about 2 mM to about 20 mM ammonium acetate, inclusive, or from about 5 mM to about 15 mM ammonium acetate, and from about 1 mM to 10 mM, or from about 2 mM to about 5 mM N-methylmorpholine (NMM). The pH of each diluted mobile phase concentrate in a two part buffer system may be about pH 5.2 and about pH 10.2, respectively.

The user may be instructed to flow the sample with the diluted mobile phase or ready-to-use mobile phase through the column to substantially resolve and retain the at least one biomolecule (e.g., protein such as a mAb). In addition, the instructions instruct the user to detect at least one biomolecule (e.g., protein such as a mAb) using a detector.

Stability studies indicate acceptable shelf life of 10X buffer system concentrate of at least 6 months, at least 12 months, at least 18 months, or more, when stored factory sealed at refrigerated temperature about 4 °C. Optionally, an organic solvent such as acetonitrile or isopropanol may be employed at a concentration of up to 10% v/v to extend shelf life. Stability studies indicate acceptable shelf life of ready-to-use buffer of at least 3 months, at least 6 months, at least 12 months, or more, when stored factory sealed at refrigerated temperature about 4 °C. A preliminary shelf life study of the buffer at room temperature has been conducted and showed promising factory sealed shelf life results of at least 3 months, at least 6 months, at least 12 months or more, of ready-to-use and 10X buffer system concentrate at room temperature. Room temp shelf life of ready to use buffer is at least 1 month with proper vessel to minimize microbial growth (incoming air is filtered).

### Uses

A method of separating an analyte from a sample is provided, the method comprising flowing a mobile phase through a chromatography column, wherein the mobile phase comprises a 2 part aqueous buffer system, wherein part A of the aqueous buffer system comprises from about 10-25 mM ammonium acetate, 3-10 mM N-methylmorpholine, and a pH 4.5-5.5 adjusted with acetic acid; and part B of the aqueous buffer system comprises 2-10 mM ammonium acetate, 1-5 mM N-methyl morpholine, and is a pH 9.5 to about 10.5 adjusted with ammonium hydroxide; injecting a sample comprising the analyte into the mobile phase; and eluting the analyte from the column. The method may further include detecting the analyte in the eluate. The method may further include washing the column after injecting prior to eluting the column. The method may further comprise determining the molecular weight of the analyte. The method may further comprise detecting the analyte with a mass spectrometer. The method may further comprise generating analyte ions. The method may further comprise acquiring a mass spectrum of the analyte ions.

In some embodiments, part A of the aqueous buffer system comprises about 15 mM ammonium acetate, about 5 mM N-methylmorpholine, adjusted to pH 5.2 with acetic acid.

In some embodiments, part B of the aqueous buffer system comprises about 5 mM ammonium acetate, about 2 mM N-methylmorpholine, adjusted to pH 10.2 with ammonium hydroxide.

In embodiments, the ammonium acetate, acetic acid, ammonium hydroxide, and N-methylmorpholine may each contain no more than about 100 ppb of an individual metal impurity.

The analyte may be a protein. The protein may be an antibody. The protein may be selected from the group consisting of a monoclonal antibody, an antigen-binding fragment of an antibody, isolated protein, synthetic protein, and a recombinant protein.

In some embodiments, the protein analyte may be eluted from the column using a gradient elution or step isocratic elution. The gradient elution may be pH gradient elution. The pH gradient elution may be selected from a linear gradient, segmented gradient, curved gradient, step gradient, and the like. A linear pH gradient may comprise a linear change in mobile phase pH from lower pH to higher pH over the course of performing a chromatographic separation. A step pH gradient may comprise a stepwise change in mobile phase pH over the course of a chromatographic separation. A segmented pH gradient may comprise two or more linear gradients of different slopes from lower pH to higher pH over the course of a chromatographic separation. A curved pH gradient may comprise a convex or concave curved pH gradient over the course of the chromatographic separation. The gradient elution may be a linear gradient. The linear gradient may be a linear pH gradient. The gradient may comprise a slope within a range of 0.1-10 %B/CV, or 0.5-5% B/column volume (CV) of the eluate. The gradient may comprise a slope within a range of 1-3% B/column volume (CV) of the eluate.

The chromatography column may be a liquid chromatography column. The chromatography column may comprise an ion exchange resin. The chromatography column may comprise a size-exclusion chromatography resin. The ion exchange resin may be a cation exchange resin. The cation exchange resin may be a strong cation exchange resin or a weak cation exchange resin.

Results in examples 2 and 3 have been reproduced at a contract facility (Intertek) using different equipment (Thermo Orbitrap MS vs Sciex X500B MS).

### EXAMPLES

### Example 1. Buffer Preparation

In this example, a new buffer system was developed for reproducible, MS-compatible chromatographic methods, for example, for pH gradient elution using cation-exchange chromatography. All materials were 99.99+% purity on a trace metals basis. Total metallic impurities < 100 ppb. Trace metal reagents are critical to reduce metal adducts observed in MS.

A standard ammonium acetate buffer system was optimized for use as a comparative buffer system. Compared to prior art, the concentration of ammonium acetate (NH₄Ac) in the comparative standard buffer was decreased in order to optimize MS compatibility: NH₄Ac concentration was decreased to no more than about 20 mM NH4Ac compared to prior art pH gradient buffer systems having 40-50 mM NH₄Ac. The comparative optimized standard buffer (ammonium acetate only) was prepared as follows. Buffer A = 20mM NH₄Ac adjusted to pH 5.2 with acetic acid. Buffer B = 5mM NH₄Ac adjusted to pH 10.2 with ammonium hydroxide. The comparative optimized standard NH₄Ac only buffer system was found to be stable for at least about four weeks at room temperature (under inlet air filter). The optimized ammonium acetate only comparative buffer may be utilized to achieve good separation of low pI mAbs, but its use requires significant optimization (very shallow gradient) and slope adjustments day to day (sensitive to very minor changes in buffer pH), and suffers from batch to batch variability.

The new pH gradient buffer system employing ammonium acetate and N-methylmorpholine was prepared as follows. The new buffer system 1X Buffer A = 15mM NH₄Ac + 5mM N-methylmorpholine adjusted to pH 5.2 with acetic acid; and 1X Buffer B = 5mM NH₄Ac + 2mM N-methylmorpholine adjusted to pH 10.2 with ammonium hydroxide. Procedure: Dissolve ammonium acetate in 1L MS grade water, add N-methylmorpholine and mix then adjust to pH 5.2 with acetic acid. Dissolve ammonium acetate in 1L MS grade water, add N-methylmorpholine and mix then adjust to pH 10.2 with ammonium hydroxide. A pH probe was employed for pH adjustment. Preferably, the pH probe should not be immersed in the bulk buffer solution to avoid contaminating the buffer with pH probe salts. Instead, the pH of an aliquot should be tested during pH adjustment. The new buffer comprising ammonium acetate and NMM gives reproducible performance with steeper, more practical gradients, as well as very shallow gradients, and does not vary day to day, or batch to batch. Robustness and ease of use when performing native MS are major issues the new NMM buffer resolves. The new buffer comprising ammonium acetate and NMM is suitable for both UV and MS detection.

The new 1X buffer system was found to be stable for at least about 4 weeks at room temperature (inlet air filter). The inlet air filter prevents or greatly reduces ingress of microbial contamination from the lab atmosphere. The buffer systems employ deionized water using LC/MS grade water, MS grade water, or Type 1 reagent grade water with a specific resistance of 18.2 megohm-cm filtered through a 0.2 micron filter. The water may have trace impurities ≤20ppb.

A 10X buffer system is prepared for dilution prior to use. Buffer A = 150mM NH₄Ac + 50mM N-methylmorpholine adjusted to pH 5.2 with acetic acid. Buffer B = 50mM NH₄Ac + 20mM N-methylmorpholine adjusted to pH 10.2 with ammonium hydroxide. Procedure: Dissolve ammonium acetate in 1L MS grade water, add N-methylmorpholine and mix then adjust to pH 5.2 with acetic acid. Dissolve ammonium acetate in 1L MS grade water, add N-methylmorpholine and mix then adjust to pH 10.2 with ammonium hydroxide. A pH probe is employed for pH adjustment.

Eluent solutions may be prepared from the 10X by diluting the 10X Buffer A and B ten-fold with deionized water (wt/wt or vol/vol) using LC/MS grade water, MS grade water, or Type 1 reagent grade water with a specific resistance of around18.2 megohm-cm filtered through a 0.2 micron filter. The water may have trace impurities ≤20ppb. For example, 100 mL of the 10X buffer may be diluted with 900 mL MS grade water for providing 1L of 1X buffer. With a standard benchtop pH meter, measure and record the pH of the 1X Buffer A and 1X Buffer B.

### Example 2. Chromatographic Results for Charge Variant Analysis of mAbs using Native WCX-HRMS

In this example, pH gradients were developed using the new buffer system for 5 mAbs of various pI values using a weak cation-exchange column. The mAbs are shown in Table 1A with measured pI values. In all examples, the WCX column was coupled directly to the high resolution mass spectrometer, i.e. online native HRMS. Spectra were collected in ESI+ mode across the m/z range of 2000-7000.

**Table 1A. mAbs Used for Charge Variant Analysis with pI values**

| Name | Brand Name | Subclass | Measured pI, iCIEF* (n=3) | Type | Target antigen |
|---|---|---|---|---|---|
| Infliximab | Remicade | IgG1 | 7.6 | ch | TNF |
| Cetuximab | Erbitux | IgG1 | 8.8 | ch | EGFR |
| Trastuzumab | Herceptin | IgG1 | 9.1 | hz | HER2 |
| Rituximab | Rituxan | IgG1 | 9.4 | ch | CD20 |
| NISTmab | NA | IgG1 | 9.2 | hz | NA |

| | | | | | |
|---|---|---|---|---|---|
| *Imaged capillary isoelectric focusing (iCIEF), From Goyon et al., 2017, J Chrom B, vol 1065-1066, pp 119-128, http://dx.doi.org/10.1016/j.jchromb.2017.09.033. | | | | | |

All mAb samples were 10 mg/mL including Infliximab pI 7.6 (Remicade), Cetuximab pI 8.8 (Erbitux), trastuzumab pI 9.1 (Kanjinti), NIST reference mAb pI 9.2, and Rituximab pI 9.4 (Rituxan). All five mAbs were analyzed using cation-exchange chromatography with the new buffer system. Three mAbs were tested using cation-exchange chromatography with the comparative standard buffer system, including trastuzumab-anns, rituximab-abbs, and NIST reference mAb. The pH gradients were optimized for each mAb, starting from exemplary pH gradients shown in Table 1B and Table 2.

The LC system was an Agilent 1290 Infinity II UHPLC system: Binary pump: G7120A (JetWeaver V35); Multi sampler: G7167B (20uL loop); Multicolumn Thermostat: G7116B; and DAD: G7117B. The mass spectrometer was a Sciex X500B quadrapole time-of-flight mass spectrometer operated in positive ion electrospray mode (ESI+) The mass range acquired was m/z 2000-7000.

The weak cation exchange column was Phenomenex^{®} bioZen 6 µm WCX, 150 x 2.1mm, monosized polymeric non-porous particle [-CH₂CH₂CH(CO₂⁻)-CH(CO₂⁻)-CH₂-].

The chromatographic conditions included (Fast Equil Method); Flow: 0.3 mL/min;_Temp: 30°C; Inj Vol: 10 uL (100ug on-column). A first exemplary pH Gradient used for New Buffer comprises 20-50% B over 15 min (slope = 1.2%B/CV), as shown in Table 1B . The flow rate during column equilibration is increased to 0.6 mL/min for fast equilibration.

The new buffer system included buffer A = 15mM NH₄Ac + 5mM N-methylmorpholine, pH 5.2 with acetic acid (HAc); and buffer B = 5mM NH₄Ac + 2mM N-methylmorpholine (NMM), pH 10.2 with ammonium hydroxide (NH₄OH).

The comparative optimized standard buffer system included buffer A = 20mM NH4Ac, pH 5.2 with HAc, and buffer B = 5mM NH₄Ac, pH 10.2 with NH₄OH. As mentioned above, the amount of ammonium acetate (NH4Ac) in the optimized comparative standard buffer system was significantly reduced compared to prior art literature references.

**Table 1B. pH Gradient for New Buffer System 20-50%B (slope = 1.2%B/CV)**

| Time (min) | %A | %B | Flow (mL/min) |
|---|---|---|---|
| 0.0 | 80 | 20 | 0.3 |
| 1.0 | 80 | 20 | 0.3 |
| 16.0 | 50 | 50 | 0.3 |
| 16.01 | 0 | 100 | 0.3 |
| 18.6 | 0 | 100 | 0.3 |
| 18.8 | 100 | 0 | 0.6 |
| 19.05 | 100 | 0 | 0.6 |
| 19.06 | 80 | 20 | 0.6 |
| 20.80 | 80 | 20 | 0.6 |
| 20.9 | 80 | 20 | 0.3 |
| 21.0 | 80 | 20 | 0.3 |

A second exemplary pH Gradient using comparative Standard Buffer comprising 20-50% B over 15 min (slope = 1.2%B/CV) is shown in Table 2. The standard buffer system included buffer A = 20mM NH₄Ac, adjusted to pH 5.2 with HAc; and buffer B = 5mM NH₄Ac, adjusted to pH 10.2 with NH₄OH.

**Table 2. pH Gradient for Standard Buffer 20-50%B (slope = 1.2%B/CV)**

| Time (min) | %A | %B | Flow (mL/min) |
|---|---|---|---|
| 0.0 | 80 | 20 | 0.3 |
| 1.0 | 80 | 20 | 0.3 |
| 16.0 | 50 | 50 | 0.3 |
| 16.01 | 0 | 100 | 0.3 |
| 18.6 | 0 | 100 | 0.3 |
| 18.8 | 100 | 0 | 0.6 |
| 19.05 | 100 | 0 | 0.6 |
| 19.06 | 80 | 20 | 0.6 |
| 22.35 | 80 | 20 | 0.6 |
| 22.36 | 80 | 20 | 0.3 |
| 22.5 | 80 | 20 | 0.3 |

### Chromatographic results

### Example 2A. WCX-HRMS of Trastuzumab-anns

Monoclonal antibody Trastuzumab-anns (Kajinti, 10 mg/mL) having pI 9.1 was subjected to native WCX-HRMS using the New Buffer; 10 uL (100 ug) was injected on a Phenomenex^{®} bioZen 6µm WCX, 150 x 2.1mm weak cation exchange column, using a 30-60%B gradient over15 min according to Table 3, +TOF MS (2000-7000). The HRMS total ion current (TIC) chromatogram from Trastuzumab-anns (Kanjinti) with the Phenomenex^{®} bioZen WCX weak cation exchange column using new buffer system with NH₄Ac + NMM is shown in FIG. 1A.

**Table 3. pH gradient - 30-60% B**

| Time (min) | %A | %B | Flow (mL/min) | Notes |
|---|---|---|---|---|
| 0.0 | 70 | 30 | 0.3 | |
| 1.0 | 70 | 30 | 0.3 | |
| 16.0 | 40 | 60 | 0.3 | |
| 16.01 | 0 | 100 | 0.3 | |
| 18.6 | 0 | 100 | 0.3 | Divert valve to waste |
| 18.8 | 100 | 0 | 0.6 | |
| 19.05 | 100 | 0 | 0.6 | |
| 19.06 | 70 | 30 | 0.6 | |
| 20.80 | 70 | 30 | 0.6 | |
| 20.9 | 70 | 30 | 0.3 | Divert valve to source |
| 21.0 | 70 | 30 | 0.3 | |

Monoclonal antibody Trastuzumab-anns (Kajinti, 10 mg/mL) having pI 9.1 was subjected to native WCX-HRMS using the optimized standard buffer using a gradient of 20-50%B over 15 min according to Table 2; 10 uL (100 ug) was injected onto a Phenomenex^{®} bioZen 6µm WCX, 150 x 2.1mm weak cation exchange column according to Table 2, +TOF MS (2000-7000). The total ion current (TIC) chromatogram for Trastuzumab-anns (Kanjinti) with the bioZen WCX column using the standard buffer system NH₄Ac without NMM is shown in FIG. 1B.

### Example 2B. WCX-HRMS of Rituximab-abbs

Monoclonal antibody Rituximab-abbs (Rituxan) (10 mg/mL) having pI 9.4 was subjected to native WCX-HRMS using the New Buffer. A 10 uL (100 ug) aliquot was injected onto a Phenomenex^{®} bioZen6µm WCX, 150 x 2.1mm weak cation exchange column and chromatographed using a 65-100%B gradient over15 min , +TOF MS (2000-7000) as shown in Table 4. The total ion current (TIC) chromatogram for Rituximab-abbs (Rituxan) on the bioZen WCX column using the new buffer system with NH₄Ac + NMM is shown in FIG. 2A.

**Table 4. pH gradient -60-100%B**

| Time (min) | %A | %B | Flow (mL/min) | Notes |
|---|---|---|---|---|
| 0.0 | 40 | 60 | 0.3 | |
| 1.0 | 40 | 60 | 0.3 | |
| 16.0 | 0 | 100 | 0.3 | |
| 16.01 | 0 | 100 | 0.3 | |
| 18.6 | 0 | 100 | 0.3 | Divert valve to waste |
| 18.8 | 100 | 0 | 0.6 | |
| 19.05 | 100 | 0 | 0.6 | |
| 19.06 | 40 | 60 | 0.6 | |
| 20.80 | 40 | 60 | 0.6 | |
| 20.9 | 40 | 60 | 0.3 | Divert valve to source |
| 21.0 | 40 | 60 | 0.3 | |

Monoclonal antibody Rituximab-abbs (Rituxan, 10 mg/mL) having pI 9.4 was subjected to native WCX-HRMS using the optimized standard buffer with a 60-100%B gradient over 15 min as shown in Table 4; 10 uL (100 ug) was injected onto a Phenomenex^{®} bioZen 6µm WCX, 150 x 2.1mm weak cation exchange column, +TOF MS (2000-7000). The total ion current (TIC) chromatogram for Rituximab-abbs (Rituxan) on the bioZen WCX column using the standard buffer system NH₄Ac without NMM is shown in FIG. 2B.

### Example 2C. WCX-HRMS of NIST Reference mAb

Monoclonal antibody NIST Reference mAb (10 mg/mL) having pI 9.2 was subjected to native WCX-HRMS using the New Buffer; 10 uL (100 ug) was injected onto a Phenomenex^{®} bioZen 6µm WCX, 150 x 2.1mm weak cation exchange column, using a gradient of 65-100%B over 15 minutes, +TOF MS (2000-7000). The total ion current (TIC) chromatogram for NIST Reference mAb on the bioZen WCX column using the new buffer system with NH₄Ac + NMM is shown in FIG. 3A.

Monoclonal antibody NIST Reference mAb having pI 9.2 was subjected to native WCX-HRMS using the optimized standard buffer and 60-100%B gradient over 15 min as shown in Table 4; 10 uL (100 ug) was injected onto a Phenomenex^{®} bioZen 6µm WCX 150 x 2.1mm weak cation exchange column, using the gradient shown in Table 4, +TOF MS (2000-7000). The total ion current (TIC) chromatogram for NIST mAb on a bioZen WCX column using the standard buffer system NH₄Ac without NMM is shown in FIG. 3B.

### Example 2D. WCX-HRMS of Infliximab

Monoclonal antibody Infliximab (Remicade, 10 mg/mL) having pI 7.6 was subjected to native WCX-HRMS using the New Buffer and a gradient of 20-35%B over 15 min according to Table 5; 10 uL (100 ug) was injected onto a Phenomenex^{®} bioZen 6µm WCX 150 x 2.1mm weak cation exchange column, +TOF MS (2000-7000). The total ion current (TIC) chromatogram for Infliximab on the bioZen WCX column using the new buffer system with NH₄Ac + NMM is shown in FIG. 4.

**Table 5. pH Gradient - 20-35%B**

| Time (min) | %A | %B | Flow (mL/min) | Notes |
|---|---|---|---|---|
| 0.0 | 80 | 20 | 0.3 | |
| 1.0 | 80 | 20 | 0.3 | |
| 16.0 | 65 | 35 | 0.3 | |
| 16.01 | 0 | 100 | 0.3 | |
| 18.6 | 0 | 100 | 0.3 | Divert valve to waste |
| 18.8 | 100 | 0 | 0.6 | |
| 19.05 | 100 | 0 | 0.6 | |
| 19.06 | 80 | 20 | 0.6 | |
| 20.80 | 80 | 20 | 0.6 | |
| 20.9 | 80 | 20 | 0.3 | Divert valve to source |
| 21.0 | 80 | 20 | 0.3 | |

### Example 2E. WCX-HRMS of Cetuximab

Monoclonal antibody Cetuximab (Erbitux; 10 mg/mL) having pI 8.8 was subjected to native WCX-HRMS using the New Buffer and a gradient of 25-55%B over 15 min according to Table 6; 10 uL (100 ug) was injected onto Phenomenex^{®} bioZen 6µm WCX, 150 x 2.1mm weak cation exchange column, +TOF MS (2000-7000). The total ion current (TIC) chromatogram for Cetuximab on the bioZen WCX column using the new buffer system with NH₄Ac + NMM is shown in FIG. 5.

**Table 6. pH Gradient - 25-55%B**

| Time (min) | %A | %B | Flow (mL/min) | Notes |
|---|---|---|---|---|
| 0.0 | 75 | 25 | 0.3 | |
| 1.0 | 75 | 25 | 0.3 | |
| 16.0 | 45 | 55 | 0.3 | |
| 16.01 | 0 | 100 | 0.3 | |
| 18.6 | 0 | 100 | 0.3 | Divert valve to waste |
| 18.8 | 100 | 0 | 0.6 | |
| 19.05 | 100 | 0 | 0.6 | |
| 19.06 | 75 | 25 | 0.6 | |
| 20.80 | 75 | 25 | 0.6 | |
| 20.9 | 75 | 25 | 0.3 | Divert valve to source |
| 21.0 | 75 | 25 | 0.3 | |

### Example 3. SCX-HRMS Data using New MS Compatible Buffer for Improved pH Gradients

In this example, methods were developed for analysis of five monoclonal antibodies and charge variants thereof by strong cation exchange liquid chromatography (SCX) directly coupled to high-resolution mass spectrometry (HRMS) using pH gradient elution with the new buffer system (ammonium acetate + N-methylmorpholine), or pH gradient elution with the optimized standard buffer system (ammonium acetate only).

Five different mAbs were tested including Infliximab pI 7.6 (Remicade), Cetuximab pI 8.8 (Erbitux), trastuzumab pI 9.1 (Kanjinti), NIST reference mAb pI 9.2, and Rituximab pI 9.4 (Rituxan).

The new buffer system was Buffer A = 15mM NH₄Ac + 5mM N-methylmorpholine adjusted to pH 5.2 with acetic acid. Buffer B = 5mM NH₄Ac + 2mM N-methylmorpholine adjusted to pH 10.2 with ammonium hydroxide.

The comparative optimized standard buffer system was Buffer A = 20mM NH₄Ac adjusted to pH 5.2 with acetic acid. Buffer B = 5mM NH₄Ac adjusted to pH 10.2 with ammonium hydroxide

Both buffer systems were prepared according to example 1 unless otherwise specified.

The strong cation exchange (SCX) column was an experimental 3 um SCX, 100 x 2.1 mm, prepared by Phenomenex R&D.

The LC column eluate was monitored using UV absorbance at 280 nm, or HRMS total ion current (TIC) chromatogram. Raw MS spectral data for new and standard buffer systems were compared as follows.

Results shown in this example demonstrate that MS signal intensities and spectral resolution using the new buffer are similar to those observed previously with the optimized standard pH gradient. However, the most intense MS peaks with new buffer system (NH4Ac + NMM) are shifted to higher masses/lower charge states (+22 to +27 with the new buffer vs +25 to +30 with the optimized standard buffer) compared to the optimized standard buffer system (NH4Ac only). This phenomenon is seen for all mAbs tested, including Trastuzumab (FIG. 6E vs. FIG. 6F), Infliximab (FIG. 7E vs FIG. 7F), Cetuximab (FIG. 8E vs FIG. 8F), Rituximab (FIG. 9E vs FIG. 9F), and NIST mAb (FIG. 10E vs FIG 10F). This phenomenon was also seen using WCX columns (data not shown). Using optimized standard buffer, raw MS data for NIST mAb in WCX-HRMS (FIG. 11) shows most intense peaks at +25 to +30 similar to raw MS data for NIST mAb in SCX-HRMS (FIG. 10F). Generally, higher masses/lower charge states are preferred since the mass differences that can be differentiated in the reconstructed intact mass spectra are improved with lower charge (smaller denominator).

System: Agilent 1290 Infinity II UHPLC system using
Binary pump: G7120A (JetWeaver V35);
Multisampler: G7167B (20uL loop);
Multicolumn Thermostat: G7116B;
DAD: G7117B;
+TOF MS (2000-7000).
   Conditions: Flow: 0.3mL/min;
   Temp: 30°C;
   Detection: UV @ 280nm;
Inj Vol: 4 uL (40ug on-column), or 10uL (100ug on column for NIST mAb and Cetuximab).

Gradient: The %B spanned and thus the pH gradient was optimized for each mAb using the experimental 3um SCX, 100 x 2.1mm column pH Gradient (slope = 1.2%B/CV, 5.6CV equil). Table 7 shows an exemplary pH gradient of 35-65%B/10 min.

**Table 7. pH gradient-35-65%B**

| Time (min) | %A | %B | Flow (mL/min) |
|---|---|---|---|
| 0.0 | 65 | 35 | 0.3 |
| 0.5 | 65 | 35 | 0.3 |
| 10.5 | 35 | 65 | 0.3 |
| 10.51 | 0 | 100 | 0.3 |
| 12.22 | 0 | 100 | 0.3 |
| 12.23 | 65 | 35 | 0.3 |
| 14.5 | 65 | 35 | 0.3 |

### Example 3A. SCX-HRMS of Trastuzumab

SCX-HRMS of Trastuzumab-anns (Kanjinti) was performed.

Trastuzumab-anns (Kanjinti) (10 mg/mL; 4 uL inj) was injected onto a strong cation exchange column (experimental 3 µm SCX, 100 x 2.1mm) using the new buffer system containing NH₄Ac + NMM and a pH gradient of 35-65% B over 10 min at flow rate of 0.3 mL/min. A UV chromatogram monitored by Abs 280 nm is shown in FIG. 6A. The major peak eluted at 5.784 min RT.

FIG. 6B shows a total ion current (TIC) chromatogram from the SCX-HRMS analysis of Trastuzumab-anns (4uL inj, 10 mg/mL) using the strong cation exchange LC column (experimental 3 µm SCX, 100 x 2. Imm) with the new buffer system containing NH₄Ac + NMM and a pH gradient of *35-65%* B over 10 min at flow rate of 0.3 mL/min, 30 °C; +TOF (2000-7000), MS temp 500 °C. The dotted line forming a box within the major peak at 4.83 min RT illustrates the approximate area for MS analysis as shown in FIG. 6D and FIG. 6E.

FIG. 6C shows a total ion current (TIC) chromatogram from SCX-HRMS of Trastuzumab-anns (4uL inj, 10 mg/mL) using the strong cation exchange LC column (experimental 3 µm SCX, 100 x 2.1mm) with the standard buffer system containing NH₄Ac only and a pH gradient of 20-50% B over10 min, 30 °C; +TOFMS (2000-7000), MS temp 500 °C. The dotted line forming a box within the major peak at 5.09 min RT illustrates the approximate area for MS analysis shown in FIG. 6F and FIG. 6G.

FIG. 6D shows raw MS spectrum from SCX-HRMS of Trastuzumabanns (4ul inj, 10 mg/mL) using the strong cation exchange column (experimental 3 µm SCX, 100 x 2.1mm) with the new buffer system containing NH₄Ac + NMM and a pH gradient of 35-65% B over10 min, +TOFMS (2000-7000) from 4.909 to 5.131 min. The mass/charge (m/z) range of 2000 to 7000 Da is shown.

FIG. 6E shows raw MS spectrum from SCX-HRMS of Trastuzumabanns (4ul inj, 10 mg/mL) having pI 9.1, from 4.858 to 5.217 min, using the strong cation exchange LC column (experimental 3 µm SCX, 100 x 2.1mm) with new buffer system containing NH₄Ac + NMM and a pH gradient of 35-65% B over10 min, +TOFMS (2000-7000). The m/z range displayed was narrowed to those giving the highest intensities for the native analyte (~4800-6600 Da) with this buffer system. Additionally, the charge of the analyte ions in the charge state envelope are indicated. With the new buffer system, the most intense MS peaks appear at higher masses/lower charges (+22 to +27) than seen with optimized standard NH₄Ac buffer system (+25 to +30), as shown in FIG. 6F. The same mass range is used for both spectra, 6E and 6F.

FIG. 6F shows raw MS spectrum from SCX-HRMS of Trastuzumabanns (4ul inj, 10 mg/mL) having pI 9.1, from 4.960 to 5.217 min, using strong cation exchange LC column (experimental 3 µm SCX, 100 x 2.1mm) with standard buffer system containing NH₄Ac with no NMM and a pH gradient of 20-50% B/10 min, +TOFMS (2000-7000) . The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH₄Ac + NMM buffer system (+22 to +27), as shown in FIG. 6E.

FIG. 6G shows raw MS spectrum from SCX-HRMS of Trastuzumabanns (4ul, 10 mg/mL) having pI 9.1 using strong cation exchange column (experimental 3 µm SCX, 100 x 2.1mm) with optimized standard buffer system containing NH₄Ac with no NMM and a pH gradient of 20-50% B/10 min, +TOF(2000-7000) from 4.960 to 5.217 min. Mass/charge range from 2000 to 7000 Da is shown.

### Example 3B. SCX-HRMS of Infliximab

SCX-HRMS of Infliximab (Remicade) having pI 7.6 was performed.

FIG. 7A shows a UV chromatogram monitored by Abs 280 nm from the SCX-UV analysis of Infliximab (10 mg/mL; 4 uL inj) using strong cation exchange column (experimental 3 µm SCX, 100 x 2.1mm) with new buffer system containing NH₄Ac + NMM and a pH gradient of 20-35% B over10 min at flow rate of 0.3 mL/min. Good separation of variants was observed using a pH gradient with new buffer system: five distinct peaks were observed eluting at 3.357,4.249, 5.018, 5.759, and 6.563 min RT.

FIG. 7B shows a total ion current (TIC) chromatogram from SCX-HRMS of Infliximab (4ul, 5 mg/mL) using a strong cation exchange LC column (experimental 3 µm SCX, 100 x 2.1mm) with new buffer system containing NH₄Ac + NMM and a pH gradient of 20-35% B over 10 min at flow rate of 0.3 mL/min, 30 °C; +TOF(2000-7000), MS temp 500 °C. Good separation of variants was observed using pH gradient with new buffer system: five distinct peaks were observed eluting at 6.07, 6.57, 7.17, 7.87, and 8.57 min RT. The dotted line forming a box within the major peak at 8.57 min RT illustrates the approximate area for MS analysis as shown in FIG. 7D and FIG. 7E.

FIG. 7C shows a total ion current (TIC) chromatogram from SCX-HRMS of Infliximab (4ul, 5 mg/mL) using a strong cation exchange LC column (experimental 3 µm SCX, 100 x 2.1mm) with standard buffer system containing NH₄Ac only and a pH gradient of 15-25% B over 10 min, 30 °C; +TOF(2000-7000), MS temp 500 °C. The dotted line forming a box within the major peak at 5.63 min RT illustrates the approximate area for MS analysis shown in FIG. 7F and FIG. 7G. Very poor separation of charge variants was given with the standard buffer for this low pI mAb (pI = 7.6). Comparison of this figure with the previous one (Figure 7B) demonstrates the performance benefit obtained by having good pH control at lower pHs than those routinely possible with the standard buffer.

FIG. 7D shows raw MS spectrum from SCX-HRMS of Infliximab (4ul, 5 mg/mL) using a strong cation exchange column (experimental 3 µm SCX, 100 x 2.1mm) with new buffer system containing NH₄Ac + NMM and a pH gradient of 20-35% B/10 min, +TOF(2000-7000) from 8.621 to 9.032 min. Mass/charge range from 2000 to 7000 Da is shown.

FIG. 7E shows raw MS spectrum from SCX-HRMS of Infliximab (4ul, 5 mg/mL) from 8.621 to 9.032 min, using strong cation exchange LC column (experimental 3 µm SCX, 100 x 2.1mm) with new buffer system containing NH₄Ac + NMM and a pH gradient of 20-35% B over 10 min, +TOF(2000-7000) The m/z range displayed was narrowed to that giving the highest intensities for the native analyte (~4800-6900 Da) with this buffer system. Additionally, the charge of the analyte ions in the charge state envelope are indicated. As shown in FIG. 7E, with the new buffer system, the most intense MS peaks appear at higher masses/lower charges (+22 to +27) than seen with optimized standard NH₄Ac buffer system (+25 to +30), shown in FIG. 7F.

FIG. 7F shows raw MS spectrum from SCX-HRMS of Infliximab (4ul, 5 mg/mL), from 5.593 to 5.730 min, using strong cation exchange LC column (experimental 3 µm SCX, 100 x 2.1mm) with standard buffer system containing NH₄Ac with no NMM and a pH gradient of 15-25% B over 10 min, +TOF(2000-7000). The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH₄Ac + NMM buffer system (+22 to +27), as shown in FIG. 7E.

FIG. 7G shows raw MS spectrum from SCX-HRMS of Infliximab (4ul, 5 mg/mL), using strong cation exchange column (experimental 3 µm SCX, 100 x 2.1mm) with optimized standard buffer system containing NH₄Ac with no NMM and a pH gradient of 15-25% B/10 min, +TOF(2000-7000) from 5.559 to 5.696 min. Mass/charge range from 2000 to 6900 Da is shown.

### Example 3C. SCX-HRMS of Cetuximab

SCX-HRMS of Cetuximab having pI 8.8 was performed.

FIG. 8A shows a UV chromatogram by Abs 280 nm from SCX-UV analysis of Cetuximab (4 mg/mL) using a strong cation exchange column (experimental 3 µm SCX, 100 x 2.1mm) with new buffer system containing NH₄Ac + NMM and a pH gradient of 30-60% B over 10 min at flow rate of 0.3 mL/min. Multiple distinct variant peaks were observed, e.g., at 3.65, 4.03, 4.58, 5.20, and 5.93 min RT.

FIG. 8B shows a total ion current (TIC) chromatogram from SCX-HRMS of Cetuximab (5 mg/mL; 10 uL inj) using a strong cation exchange LC column (experimental 3 µm SCX, 100 x 2.1mm) with the new buffer system containing NH₄Ac + NMM and a pH gradient of 30-60% B over10 min at flow rate of 0.3 mL/min, 30 °C; +TOF(2000-7000), MS temp 500 °C. Multiple distinct variant peaks were observed at 3.33, 3.92, 4.54, and 5.22 min RT. The dotted line forming a box within the major peak at 4.54 min RT illustrates the approximate area for MS analysis as shown in FIG. 8D and FIG. 8E.

FIG. 8C shows a total ion current (TIC) chromatogram from the SCX-HRMS analysis of Cetuximab (5 mg/mL; 10 uL inj) using a strong cation exchange LC column (experimental 3 µm SCX, 100 x 2.1mm) with standard buffer system containing NH₄Ac only and a pH gradient of 17-40% B/10 min, 30 °C; +TOF(2000-7000), MS temp 500 °C. The dotted line forming a box within the major peak at 4.68 min RT illustrates the approximate area for MS analysis shown in FIG. 8F and FIG. 8G.

FIG. 8D shows a raw MS spectrum from SCX-HRMS of Cetuximab (5 mg/mL; 10 uL inj) using a strong cation exchange column (experimental 3 µm SCX, 100 x 2.1mm) with new buffer system containing NH₄Ac + NMM and a pH gradient of 30-60% B over 10 min, +TOF(2000-7000) from 4.601 to 9.032 min. Mass/charge range from 2000 to 7000 Da is shown.

FIG. 8E shows a raw MS spectrum from SCX-HRMS of Cetuximab (5 mg/mL; 10 uL inj) from 4.601 to 4.892 min, using a strong cation exchange LC column (experimental 3 µm SCX, 100 x 2.1mm) with new buffer system containing NH₄Ac + NMM and a pH gradient of 30-60% B over 10 min, +TOF(2000-7000). The m/z range displayed was narrowed to that giving the highest intensities for the native analyte (~4800-6900 Da) with this buffer system. Additionally, the charge of the analyte ions in the charge state envelope are indicated. As shown in FIG. 8E, when using pH gradient elution with the new buffer system, the most intense MS peaks appear at higher masses/lower charges (+22 to +27) than seen with optimized standard NH₄Ac buffer system (+25 to +30), as shown in FIG. 8F.

FIG. 8F shows raw MS spectrum from SCX-HRMS of Cetuximab (5 mg/mL; 10 uL inj), from 4.533 to 4.892 min, using a strong cation exchange LC column (experimental 3 µm SCX, 100 x 2.1mm) with the standard buffer system containing NH₄Ac with no NMM and a pH gradient of 17-40% B over10 min,+TOF(2000-7000). The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at lower masses/higher charges (+25 to +30), when compared to new NH₄Ac + NMM buffer system (+22 to +27), as shown in FIG. 8E.

FIG. 8G shows a raw MS spectrum from SCX-HRMS of Cetuximab (5 mg/mL; 10 uL inj), from 4.533 to 4.892 min. using a strong cation exchange column (experimental 3 µm SCX, 100 x 2.1mm) with the optimized standard buffer system containing NH₄Ac with no NMM and a pH gradient of 17-40% B over 10 min, +TOF(2000-7000). Mass/charge range from 2000 to 6900 Da is shown.

### Example 3D. SCX-HRMS of Rituximab

SCX-HRMS of Rituximab (Rituxan) having pI 9.4 was performed.

FIG. 9A shows a UV chromatogram at Abs 280 nm from SCX-HRMS of Rituximab (Rituxan) having pI 9.4 (10 mg/mL) using strong cation exchange column (experimental 3 µm SCX, 100 x 2.1mm) with the new buffer system containing NH₄Ac + NMM and a pH gradient of 75-100% B over 10 min at flow rate of 0.3 mL/min. A major peak at 5.03 min RT was observed.

FIG. 9B shows a total ion current (TIC) chromatogram from the SCX-HRMS analysis of Rituximab (10 mg/mL; 4 uL inj) using strong cation exchange LC column (experimental 3 µm SCX, 100 x 2.1mm) with new buffer system containing NH₄Ac + NMM and a pH gradient of 65-100% B over 10 min at flow rate of 0.3 mL/min, 30 °C; +TOFMS (2000-7000), MS temp 500 °C. The dotted line forming a box within the major peak at 5.88 min RT illustrates an approximate area for MS analysis.

FIG. 9C shows a total ion current (TIC) chromatogram from the SCX-HRMS analysis of Rituximab (10 mg/mL; 4 uL inj) using a strong cation exchange LC column (experimental 3 µm SCX, 100 x 2.1mm) with standard buffer system containing NH₄Ac only and a pH gradient of 55-100% B over 10 min, 30 °C; +TOFMS (2000-7000), MS temp 500 °C. The dotted line forming a box within the major peak at 4.65 min RT illustrates the approximate area for MS analysis shown in FIG. 9F and FIG. 9G.

FIG. 9D shows a raw MS spectrum from LC-HRMS of Rituximab (10 mg/mL; 4 uL inj) using a strong cation exchange column (experimental 3 µm SCX, 100 x 2.1mm) with new buffer system containing NH₄Ac + NMM and a pH gradient of 65-100% B over 10 min, +TOFMS (2000-7000) from 6.021 to 6.363 min. Mass/charge, m/z data in a range from 2000 to 7000 Da is shown.

FIG. 9E shows a raw MS spectrum from the SCX-HRMS analysis of Rituximab (10 mg/mL; 4 uL inj) using a strong cation exchange LC column (experimental 3 µm SCX, 100 x 2.1mm) with new buffer system containing NH₄Ac + NMM and a pH gradient of 65-100% B/10 min, +TOFMS (2000-7000), from 6.021 to 6.363 min. The m/z range displayed was narrowed to that giving the highest intensities for the native analyte (~4800-6800 Da) with this buffer system. Additionally, the charge of the analyte ions in the charge state envelope are indicated. As show in FIG. 9E, when using a pH gradient with new buffer system, the most intense MS peaks appear at higher masses/lower charges (+22 to +27) than seen with optimized standard NH₄Ac buffer system (+25 to +30), shown in FIG. 9F.

FIG. 9F shows a raw MS spectrum from the SCX-HRMS analysis of Rituximab (10 mg/mL; 4 uL inj) using a strong cation exchange LC column (experimental 3 µm SCX, 100 x 2.1mm) with standard buffer system containing NH₄Ac with no NMM and a pH gradient of 55-100% B over 10 min, +TOFMS (2000-7000), from 4.550 to 4.772 min. The m/z range displayed was narrowed to that giving the highest intensities for the native analyte (~4800-6800 Da) with this buffer system. Additionally, the charge of the analyte ions in the charge state envelope are indicated. With the optimized standard buffer system, the most intense MS peaks appear at lower masses/higher charges (+25 to +30), when compared to new NH₄Ac + NMM buffer system (+22 to +27), as shown in FIG. 9E.

FIG. 9G shows a raw MS spectrum from the SCX-HRMS analysis of Rituximab (10 mg/mL; 4 uL inj) from 4.550 to 4.772 min. using a strong cation exchange column (experimental 3 µm SCX, 100 x 2.1mm) with the optimized standard buffer system containing NH₄Ac with no NMM and a pH gradient of 55-100% B/10 min, +TOFMS (2000-7000). Mass/charge range from 2000 to 7000 Da is shown.

### Example 3E. SCX-HRMS of NIST mAb

SCX-HRMS of NIST mAb having pI 9.2 was performed.

FIG. 10A shows a UV chromatogram monitored at Abs 280 nm from the SCX-UV analysis of NIST mAb having pI 9.2 (10 mg/mL) using a strong cation exchange column (experimental 3 µm SCX, 100 x 2.1mm) with the new buffer system containing NH₄Ac + NMM and a pH gradient of 95-100% B over 10 min at flow rate of 0.3 mL/min.

FIG. 10B shows a total ion current (TIC) chromatogram from SCX-HRMS of NIST mAb (10 mg/mL; 10 uL inj) using a strong cation exchange LC column (experimental 3 µm SCX, 100 x 2.1 mm) with the new buffer system containing NH₄Ac + NMM and a pH gradient of 90-100% B over 10 min at flow rate of 0.3 mL/min, 30 °C; +TOFMS (2000-7000), MS temp 500 °C. The dotted line forming a box within peak between 4.39 and 4.82 RT illustrates an approximate area for MS analysis.

FIG. 10C shows a total ion current (TIC) chromatogram from the SCX-HRMS analysis of NIST mAb (10 mg/mL; 10 uL inj) using a strong cation exchange LC column (experimental 3 µm SCX, 100 x 2.1mm) with the standard buffer system containing NH₄Ac only and a pH gradient of 80-100% B/10 min, 30 °C; +TOFMS (2000-7000), MS temp 500 °C. The dotted line forming a box within the major peak at about 4.5 min RT illustrates the approximate area for MS analysis shown in FIG. 10F and FIG. 10G.

FIG. 10D shows a raw MS spectrum from the SCX-HRMS analysis of NIST mAb (10 mg/mL; 10 uL inj) using a strong cation exchange column (experimental 3 µm SCX, 100 x 2.1mm) with the new buffer system containing NH₄Ac + NMM and a pH gradient of 90-100% B/10 min, +TOFMS(2000-7000) from 5.012 to 5.627 min. Mass/charge (m/z) data across the range from 2000 to 7000 Da is shown.

FIG. 10E shows a raw MS spectrum from the SCX-HRMS analysis of NIST mAb (10 mg/mL; 10 uL inj) using a strong cation exchange LC column (experimental 3 µm SCX, 100 x 2.1mm) with the new buffer system containing NH₄Ac + NMM and a pH gradient of 90-100% B/10 min, +TOFMS (2000-7000), from 5.012 to 5.627 min. The m/z range displayed was narrowed to that giving the highest intensities for the native analyte (~4800-6900 Da) with this buffer system. Additionally, the charge of the analyte ions in the charge state envelope are indicated. With the new buffer system, the most intense MS peaks appear at higher masses/lower charges (+22 to +27) than seen with optimized standard NH₄Ac buffer system (+25 to +30), as shown in FIG. 10F.

FIG. 10F shows a raw MS spectrum from the SCX-HRMS analysis of NIST mAb (10 mg/mL; 10 uL inj) using a strong cation exchange LC column (experimental 3 µm SCX, 100 x 2.1mm) with the standard buffer system containing NH₄Ac with no NMM and a pH gradient of 80-100% B/10 min, +TOFMS (2000-7000), from 4.259 to 4.841 min.. The m/z range displayed was narrowed to that giving the highest intensities for the native analyte (~4800-6900 Da) with this buffer system. Additionally, the charge of the analyte ions in the charge state envelope are indicated. With the optimized standard buffer system, the most intense MS peaks appear at slightly lower masses/higher charges (+25 to +30), when compared to new NH₄Ac + NMM buffer system (+22 to +27), as shown in FIG. 10E.

FIG. 10G shows a raw MS spectrum from the SCX-HRMS analysis of NIST mAb (10 mg/mL; 10 uL inj) from 4.259 to 4.841 min. using a strong cation exchange column (experimental 3 µm SCX, 100 x 2.1mm) with the optimized standard buffer system containing NH₄Ac with no NMM and a pH gradient of 80-100% B/10 min, +TOFMS (2000-7000). Mass/charge range from 2000 to 6800 Da is shown.

FIG. 11 shows a raw MS spectrum from the WCX-HRMS analysis of NIST mAb (10 mg/mL; 10 uL inj) using a weak cation exchange LC column (Phenomenex^{®} bioZen 6 µm WCX, 150 x 2.1mm) with the standard buffer system containing NH₄Ac with no NMM and a pH gradient of 60-100% B, +TOFMS (2000-7000), 500 °C, from 4.772 to 5.012 min. . The m/z range displayed was narrowed to that giving the highest intensities for the native analyte (~4800-7000 Da) with this buffer system. Additionally, the charge of the analyte ions in the charge state envelope are indicated. With the optimized standard buffer system, raw MS spectrum for NIST mAb using WCX-HRMS also exhibits the most intense MS peaks from +25 to +30, similar to SCX-HRMS raw spectrum shown in FIG. 10F.

### REFERENCES CITED

Allowable_concentrations_salt_and_buffer_components_for_MS-Harvard Center for MS-download 05-05-2021
Bailey et al., 2018, Charge variant native mass spectrometry benefits mass precision and dynamic range of monoclonal antibody intact mass analysis, MABS, vol. 10, No. 8, 1214-1225. doi.org/ 10.1080/1940862.2018.1521131.
Borman, 1987, Eluent, Effluent, Eluate, and Eluite, Anal Chem vol. 59, no. 2, Jan 15, 1987, 99A
Chen et al., 2012, Ultra-high performance liquid chromatography/tandem mass spectrometry determination of feminizing chemicals in river water, sediment and tissue pretreated using disk-type solid-phase extraction and matrix solid-phase dispersion, Talanta, Vol 89, pp. 237-245. doi.org/10.1016/j.talanta.2011.12.020
Chirita et al., 2011, Evaluation of fused-core and monolithic versus porous silica-based C18 columns and porous graphitic carbon for ion-pairing liquid chromatography analysis of catecholamines and related compounds; Volume 879, Issues 9-10, 15 March 2011, Pages 633-640; https://doi.org/10.1016/j.jchromb.2011.01.036
Fussl et al., 2018, Charge Variant Analysis of Monoclonal Antibodies Using Direct
Coupled pH Gradient Cation Exchange Chromatography to High-Resolution Native Mass Spectrometry, Anal. Chem. 90, 4669-4676. doi 10.1021/acs.analchem. 7b05241.
Fussl et al., 2019, Comprehensive characterisation of the heterogeneity of adalimumab via charge variant analysis hyphenated on-line to native high resolution Orbitrap mass spectrometry, MABS vol. 11, No. 1, 116-128, doi.org/10.1080/19420862.2018.1531664.
Goyon et al., 2017, Determination of isoelectric points and relative charge variants of 23 therapeutic monoclonal antibodies, J Chrom B, vol 1065-1066, pp 119-128, http://dx.doi.org/10.1016/j.jchromb.2017.09.033
NIST Monoclonal Antibody Reference Material 8671, 2021, https://www.nist.gov/programs-projects/nist-monoclonal-antibody-reference-material-8671
pKa Data Compiled by R. Williams-downloaded 2021-pp. 1-33
Schaefercor et al., 1996, Effect of high-performance liquid chromatography mobile phase components on sensitivity in negative atmospheric pressure chemical ionization liquid chromatography-mass spectrometry, J Am Soc Mass Spectrom, 7, 10, 1059-1069; https://doi.org/10.1016/1044-0305(96)00049-9
Nortcliffe et al., 2017, Detection-of-Intact-Antibody-Impurities-using- SCIEX X500B, Application Data Sheet, AB Sciex Document number: RUO-MKT-02-6335-A
Shukla et al., 2017, Evolving trends in mAb production processes, Bioengineering & Translational Medicine 2017: 2, 58-69, DOI 10.1002/btm2.10061

## Claims

1. A ready-to-use mobile phase composition for liquid chromatography, comprising water;
2-50 mM of an ammonium carboxylate;
1-50 mM of an N-methylmorpholine, an isomer thereof, or an analog thereof; and
a pH in the range of about pH 4.5 to about pH 10.5 at room temperature.

2. The composition of claim 1, comprising
2-25 mM of the ammonium carboxylate; and
1-10 mM of the N-methylmorpholine, the isomer thereof, or the analog thereof

3. The composition of claim 1 or claim 2, wherein the ammonium carboxylate is selected from the group consisting of ammonium acetate and ammonium formate.

4. The composition of any one of the preceding claims, wherein the N-methylmorpholine isomer is selected from the group consisting of 2-methylmorpholine, 2-methylmorpholine, 2-methyl-1,3-oxazinane, 3-methyl-1,3-oxazinane, 4-methyl-1,3-oxazinane, 5-methyl-1,3-oxazinane, and 6-methyl-1,3-oxazinane.

5. The composition of any one of the preceding claims, wherein the analog is selected from the group consisting of an alkyl C₁-C₆ morpholine, a dialkyl C₁-C₆ morpholine, an alkyl C₁-C₆-1,3-oxazinane, and a dialkylC₁-C₆-1,3-oxazinane.

6. The composition of any one of the preceding claims, wherein the mobile phase comprises a 2 part aqueous buffer system, wherein
part A of the aqueous buffer system comprises from about 10 mM to about 20mM ammonium acetate, about 3 to about 8 mM N-methylmorpholine, and a pH in the range of pH 5.0-5.5; and
part B of the aqueous buffer system comprises 2-10 mM ammonium acetate, 1-5 mM N-methyl morpholine, and a pH in the range of pH 9.5 - 10.5.

7. The composition of claim 6, wherein the pH of the part A buffer is adjusted with acetic acid.

8. The composition of claim 6 or claim 7, wherein the pH of the part B buffer is adjusted with ammonium hydroxide.

9. The composition of any one of claims 6-8, wherein the part A buffer comprises about 14 to about 16 mM of the ammonium acetate; about 4 to about 6 mM of the N-methylmorpholine; and the pH is between about pH 5.1 and about pH 5.3.

10. The composition of any one of claims 6-9, wherein the part B buffer comprises about 4 to about 6 mM of the ammonium acetate; about 1 mM to about 3 mM of the N-methylmorpholine; and the pH is between about pH 10.0 and about pH 10.4.

11. The composition of any one of claims 1 to 10, wherein the concentration of the ammonium acetate is no more than 25 mM, no more than 20 mM, or no more than about 15 mM.

12. Use of the composition of any one of claims 1 to 10 as a mobile phase in liquid chromatography, wherein the liquid chromatography further comprises a stationary phase.

13. The use of claim 12, wherein the stationary phase is selected from the group consisting of ion-exchange stationary phase, size-exclusion stationary phase, hydrophilic-interaction stationary phase, and reverse-phase stationary phase.

14. The use of claim 13, wherein the ion-exchange stationary phase is a cation exchange stationary phase.

15. Use of the composition of any one of claims 1 to 11 as a mobile phase in liquid chromatography , wherein the liquid chromatography is directly coupled (online) to a mass spectrometer.

## Patentansprüche

1. Gebrauchsfertige Zusammensetzung einer mobilen Phase für die Flüssigkeitschromatographie, umfassend
Wasser;
2-50 mM eines Ammoniumcarboxylats;
1-50 mM eines N-Methylmorpholins, eines Isomers davon oder eines Analogons davon; und
einen pH-Wert im Bereich von etwa pH 4,5 bis etwa pH 10,5 bei Raumtemperatur.

2. Zusammensetzung nach Anspruch 1, umfassend
2-25 mM des Ammoniumcarboxylats; und
1-10 mM des N-Methylmorpholins, des Isomers davon oder des Analogons davon

3. Zusammensetzung nach Anspruch 1 oder Anspruch 2, wobei das Ammoniumcarboxylat aus der Gruppe ausgewählt ist, die aus Ammoniumacetat und Ammoniumformiat besteht.

4. Zusammensetzung nach einem der vorstehenden Ansprüche, wobei das N-Methylmorpholin-Isomer aus der Gruppe ausgewählt ist, die aus 2-Methylmorpholin, 2-Methylmorpholin, 2-Methyl-1,3-oxazinan, 3-Methyl-1,3-oxazinan, 4-Methyl-1,3-oxazinan, 5-Methyl-1,3-oxazinan, und 6-Methyl-1,3-oxazinan besteht.

5. Zusammensetzung nach einem der vorstehenden Ansprüche, wobei das Analogon aus der Gruppe ausgewählt ist, die aus einem C₁-C₆-Alkylmorpholin, einem C₁-C₆-Dialkylmorpholin, einem C₁-C₆-Alkyl-1,3-oxazinan, und einem C₁-C₆-Dialkyl-1,3-oxazinan besteht.

6. Zusammensetzung nach einem der vorstehenden Ansprüche, wobei die mobile Phase ein zweiteiliges wässriges Puffersystem umfasst, wobei
Teil A des wässrigen Puffersystems etwa 10 mM bis etwa 20 mM Ammoniumacetat, etwa 3 bis etwa 8 mM N-Methylmorpholin, und einen pH-Wert im Bereich von pH 5,0-5,5 umfasst; und
Teil B des wässrigen Puffersystems 2-10 mM Ammoniumacetat, 1-5 mM N-Methylmorpholin, und einen pH-Wert im Bereich von pH 9,5-10,5 umfasst.

7. Zusammensetzung nach Anspruch 6, wobei der pH-Wert des Puffers von Teil A mit Essigsäure eingestellt wird.

8. Zusammensetzung nach Anspruch 6 oder Anspruch 7, wobei der pH-Wert des Puffers von Teil B mit Ammoniumhydroxid eingestellt wird.

9. Zusammensetzung nach einem der Ansprüche 6-8, wobei der Puffer von Teil A etwa 14 bis etwa 16 mM des Ammoniumacetats; etwa 4 bis etwa 6 mM des N-Methylmorpholins umfasst; und der pH-Wert zwischen etwa pH 5,1 und etwa pH 5,3 liegt.

10. Zusammensetzung nach einem der Ansprüche 6-9, wobei der Puffer von Teil B etwa 4 bis etwa 6 mM des Ammoniumacetats; etwa 1 mM bis etwa 3 mM des N-Methylmorpholins umfasst; und der pH-Wert zwischen etwa pH 10,0 und etwa pH 10,4 liegt.

11. Zusammensetzung nach einem der Ansprüche 1 bis 10, wobei die Konzentration des Ammoniumacetats nicht mehr als 25 mM, nicht mehr als 20 mM oder nicht mehr als etwa 15 mM beträgt.

12. Verwendung der Zusammensetzung nach einem der Ansprüche 1 bis 10 als mobile Phase in der Flüssigkeitschromatographie, wobei die Flüssigkeitschromatographie weiter eine stationäre Phase umfasst.

13. Verwendung nach Anspruch 12, wobei die stationäre Phase ausgewählt ist aus der Gruppe bestehend aus einer stationären Ionenaustausch-Phase, einer stationären Größenausschluss-Phase, einer stationären Phase für hydrophile Interaktion und einer stationären Umkehrphase.

14. Verwendung nach Anspruch 13, wobei die stationäre Ionenaustausch-Phase eine stationäre Kationenaustausch-Phase ist.

15. Verwendung der Zusammensetzung nach einem der Ansprüche 1 bis 11 als mobile Phase in der Flüssigkeitschromatographie, wobei die Flüssigkeitschromatographie direkt (online) mit einem Massenspektrometer gekoppelt ist.

## Revendications

1. Composition de phase mobile prête à l'emploi pour chromatographie en phase liquide, comprenant
de l'eau ;
2-50 mM d'un carboxylate d'ammonium ;
1-50 mM d'une N-méthylmorpholine, d'un isomère de celle-ci ou d'un analogue de celle-ci ; et
un pH dans la plage d'environ pH 4,5 à environ pH 10,5 à température ambiante.

2. Composition selon la revendication 1, comprenant
2-25 mM du carboxylate d'ammonium ; et
1-10 mM de la N-méthylmorpholine, de l'isomère de celle-ci ou de l'analogue de celle-ci.

3. Composition selon la revendication 1 ou la revendication 2, dans laquelle le carboxylate d'ammonium est sélectionné dans le groupe consistant en l'acétate d'ammonium et le formiate d'ammonium.

4. Composition selon l'une quelconque des revendications précédentes, dans laquelle l'isomère de N-méthylmorpholine est sélectionné dans le groupe consistant en la 2-méthylmorpholine, la 2-méthylmorpholine, le 2-méthyl-1,3-oxazinane, le 3-méthyl-1,3-oxazinane, le 4-méthyl-1,3-oxazinane, le 5-méthyl-1,3-oxazinane et le 6-méthyl-1,3-oxazinane.

5. Composition selon l'une quelconque des revendications précédentes, dans laquelle l'analogue est sélectionné dans le groupe consistant en une alkyl C₁-C₆ morpholine, une dialkyl C₁-C₆ morpholine, un alkyl C₁-C₆-1,3-oxazinane et un dialkyl C₁-C₆-1,3-oxazinane.

6. Composition selon l'une quelconque des revendications précédentes, dans laquelle la phase mobile comprend un système tampon aqueux en 2 parties, dans laquelle
la partie A du système tampon aqueux comprend d'environ 10 mM à environ 20 mM d'acétate d'ammonium, d'environ 3 à environ 8 mM de N-méthylmorpholine, et un pH dans la plage de pH 5,0-5,5 ; et
la partie B du système tampon aqueux comprend 2-10 mM d'acétate d'ammonium, 1-5 mM de N-méthylmorpholine, et un pH dans la plage de pH 9,5-10,5.

7. Composition selon la revendication 6, dans laquelle le pH de la partie A du tampon est ajusté avec de l'acide acétique.

8. Composition selon la revendication 6 ou la revendication 7, dans laquelle le pH de la partie B du tampon est ajusté avec de l'hydroxyde d'ammonium.

9. Composition selon l'une quelconque des revendications 6-8, dans laquelle la partie A du tampon comprend d'environ 14 à environ 16 mM de l'acétate d'ammonium ; d'environ 4 à environ 6 mM de la N-méthylmorpholine ; et le pH est entre environ pH 5,1 et environ pH 5,3.

10. Composition selon l'une quelconque des revendications 6-9, dans laquelle la partie B du tampon comprend d'environ 4 à environ 6 mM de l'acétate d'ammonium ; d'environ 1 mM à environ 3 mM de la N-méthylmorpholine ; et le pH est entre environ pH 10,0 et environ pH 10,4.

11. Composition selon l'une quelconque des revendications 1 à 10, dans laquelle la concentration en acétate d'ammonium n'est pas supérieure à 25 mM, n'est pas supérieure à 20 mM, ou n'est pas supérieure à environ 15 mM.

12. Utilisation de la composition selon l'une quelconque des revendications 1 à 10 comme phase mobile en chromatographie en phase liquide, dans laquelle la chromatographie en phase liquide comprend en outre une phase stationnaire.

13. Utilisation selon la revendication 12, dans laquelle la phase stationnaire est sélectionnée dans le groupe consistant en une phase stationnaire d'échange d'ions, une phase stationnaire d'exclusion de taille, une phase stationnaire d'interaction hydrophile et une phase stationnaire de phase inverse.

14. Utilisation selon la revendication 13, dans laquelle la phase stationnaire d'échange d'ions est une phase stationnaire d'échange de cations.

15. Utilisation de la composition selon l'une quelconque des revendications 1 à 11 comme phase mobile en chromatographie en phase liquide dans laquelle la chromatographie en phase liquide est directement couplée (en ligne) à un spectromètre de masse.
